# EUROPEAN PATENT APPLICATION

(11) **EP 4 206 227 A1**
(43) Date of publication of application: **05.07.2023**
(21) Application number: 21860493.2
(22) Date of filing: 26.08.2021
(51) Int. Cl.: C07K 16/28, C12N 15/13, C12N 15/63, A61K 39/395, A61P 35/00, A61P 37/02

(54) **ANTI-OX40 ANTIBODY, AND PHARMACEUTICAL COMPOSITION AND APPLICATION THEREOF**

(30) Priority: 26.08.2020 CN 202010872934
(71) Applicant: Shanghai Acemab Corporation Ltd., Shanghai 200131 (CN); Zhongshan Hengdong Biopharmaceutical Co., Ltd., Zhongshan, Guangdong 528437 (CN)
(72) Inventor: WONG, See Heng, Xiangtan, Hunan 411101 (CN); LI, Jianliang, Xiangtan, Hunan 411101 (CN); LI, Fubin, Chongqing Road Shanghai 200025 (CN); CAI, Wei, Xiangtan, Hunan 411101 (CN); YAN, Xiaohua, Suzhou, Jiangsu 215000 (CN); TIAN, Meng, Xiangtan, Hunan 411101 (CN); ZHANG, Junxia, Xiangtan, Hunan 411101 (CN); BI, Yanxia, Chongqing Road Shanghai 200025 (CN); WANG, Caihong, Suzhou, Jiangsu 215000 (CN)
(74) Representative: IPLodge bv
(86) International application number: PCT/CN2021/114831
(87) International publication number: WO 2022/042659

(57) **Abstract**

Provided are an anti-OX40 antibody, and a pharmaceutical composition and application thereof. The anti-OX40 antibody or an antigen-binding fragment and pharmaceutical composition thereof can be used for treating a T cell-related disease.

## Description

### TECHNICAL FIELD

This description relates to anti-OX40 antibodies, pharmaceutical composition thereof and use thereof.

### BACKGROUND

The breakthrough and development of tumor immunotherapy is a huge progress in the field of tumor treatment, and was selected as the "Breakthrough of the Year" in 2013 by "Science" magazine. There are four main approaches to harnessing the power of the immune system to fight tumors: 1) Antibodies against tumor antigens are injected into tumor patients to kill tumor cells. Part of the mechanism is that tumor cells are bound to antibodies and then killed by immune cells expressing Fc receptors; 2) Anti-tumor immune cells isolated from tumor patients are expanded in vitro, and then reinfused into the patient to exert anti-tumor effects; 3) T cells are genetically modified to express chimeric antigen receptors (CAR) that recognize tumor antigens, and then injected into tumor patients; 4) Monoclonal antibodies (such as anti-CTLA-4 and anti-PD-1/PD-L1) directed against targets of immunosuppressive signaling pathways that limit immune cell activity (known as "immune checkpoints"), which enhance the immune system's killer T cell response against tumor cells to kill tumors by blocking immunosuppressive signals.

Although the above methods have been used clinically, there is still an urgent need for new immunotherapies that work through different mechanisms in the field of tumor immunotherapy. These are because: 1) Not all tumor types respond to existing tumor immunotherapy methods; 2) Even for tumor types that respond to existing immunotherapies, not all patients having these tumor types respond to these immunotherapies; 3) Combination treatments are predicted to have stronger therapeutic effects; 4) Tumors are likely to develop mutations that escape these treatments.

Agonistic antibodies against immune co-stimulatory molecules can bind to target molecules that transmit immune activation signals on the surface of immune cells and activate the immune activation signaling pathways controlled by them, thereby enhancing the anti-tumor immune response to kill tumor cells, which is a therapy with broad prospects. Among many immune co-stimulatory molecules, OX40 is one of the earliest molecules discovered and verified to intervene in anti-tumor responses.

OX40 is a member of the TNF receptor superfamily, mainly expressed on the surface of activated T cells, including activated CD4 and CD8 T cell functional subsets, such as Th1, Th2, T_{FH}, Th17, and regulatory T cells (Tregs). OX40 has also been reported to be slightly expressed on neutrophils, NK, and NKT cells. OX40 is not expressed in naive T cells, but is specifically expressed in activated T cells. Therefore, targeting OX40 has a certain selectivity, and will not activate all T cells indiscriminately (such as targeting CD28, which is more broadly expressed). OX40L/CD252 is the natural ligand of OX40, which is mainly expressed in antigen-presenting cells such as B cells, dendritic cells (DCs), and macrophages. Expression of both OX40L and OX40 is antigen-induced, and OX40L expression is induced by CD40-CD40L signaling, Toll-like receptors (TLRs), and inflammatory cytokines. The expression of OX40 is activated by antigen-presenting cells presenting antigens to T cells to activate T cell receptor downstream signals, and is positively regulated by CD28-B7.1/2 signals, and the peak expression level occurs between 12 hours and 6 days after T cell activation. The structural analysis of OX40/OX40L supports the trimerization of OX40 and OX40L after the interaction, and the combination of OX40/OX40L is likely to be directly involved in the cell interaction. The trimerization of OX40 allows its intracellular segment to recruit TRAF2/3/5, activate NF-κB signaling, and up-regulate the expression of anti-apoptotic molecules such as Bcl-2 and Bcl-xL, thereby inhibiting apoptosis and enhancing cell survival. OX40 has also been found to cooperate with TCR signaling to affect the survival and proliferation of T cells through PI3K/Akt, and regulate the expression of cytokines such as IL-2, IL-4, IL-5 and IFN-g through NFAT. Therefore, the activation of OX40 can inhibit the apoptosis of activated T cells, promote their proliferation and produce cytokines. These are the functions of agonistic anti-OX40 that can activate OX40 signaling.

Anti-OX40 (and OX40L-Fc fusion protein) have been reported to inhibit tumor growth in a variety of tumor models, including melanoma, rectal cancer, fibrosarcoma, B-cell and T-cell lymphoma, and leukemia, including in some mice. On this basis, multiple anti-human OX40 monoclonal antibodies have entered clinical research, including Pfizer's PF04518600, Bristol-Myers Squibb's BMS-986178, and MedImmune's multiple anti-OX40 antibodies. However, the research and development of anti-OX40 as an anti-tumor drug have not progressed smoothly. Since the first clinical study was reported in 2013, the drugs of many companies have stagnated, and no antibody has yet entered the third phase of clinical research, suggesting that the immune effect pathway and design principles of anti-tumor antibodies need further research.

### SUMMARY OF DESCRIPTION

The present description provides an anti-OX40 antibody or an antigen-binding fragment thereof. The anti-OX40 antibody contains at least one CDR selected from the following sequences: SEQ ID NO: 1, SEQ ID NO:2, SEQ ID NO:3, SEQ ID NO:4, SEQ ID NO:5 and SEQ ID NO:6.

In one or more embodiments, the anti-OX40 antibody comprises HCDR1 as set forth in SEQ ID NO: 1, HCDR2 as set forth in SEQ ID NO:2, and HCDR3 as set forth in SEQ ID NO:3, and/or LCDR1 as set forth in SEQ ID NO:4, LCDR2 as set forth in SEQ ID NO:5, and LCDR3 as set forth in SEQ ID NO:6.

In one or more embodiments, the anti-OX40 antibody contains HCDR1 as shown in any of SEQ ID NO: 7-38, HCDR2 as shown in any of SEQ ID NO: 39-65, and HCDR3 as shown in any of SEQ ID NO: 66-114, and/or LCDR1 as shown in any of SEQ ID NO: 115-145, LCDR2 as shown in any of SEQ ID NO: 146-159 and LCDR3 as shown in any of SEQ ID NO: 160-199.

In one or more embodiments, the anti-OX40 antibody contains HCDR1, HCDR2 and HCDR3 shown in any one of the following groups a1 to a71:

**Table 1**

| Group | HCDR1 | HCDR2 | HCDR3 |
|---|---|---|---|
| **a1** | 23 | 57 | 79 |
| **a2** | 23 | 57 | 79 |
| **a3** | 23 | 43 | 79 |
| **a4** | 23 | 48 | 79 |
| **a5** | 34 | 50 | 99 |
| **a6** | 35 | 59 | 110 |
| **a7** | 27 | 59 | 69 |
| **a8** | 28 | 59 | 69 |
| **a9** | 32 | 56 | 105 |
| **a10** | 23 | 42 | 85 |
| **a11** | 12 | 46 | 67 |
| **a12** | 14 | 60 | 71 |
| **a13** | 34 | 52 | 98 |
| **a14** | 11 | 40 | 75 |
| **a15** | 23 | 48 | 79 |
| **a16** | 21 | 63 | 107 |
| **a17** | 23 | 57 | 79 |
| **a18** | 23 | 57 | 109 |
| **a19** | 34 | 50 | 99 |
| **a20** | 33 | 59 | 86 |
| **a21** | 38 | 43 | 107 |
| **a22** | 32 | 54 | 90 |
| **a23** | 33 | 59 | 86 |
| **a24** | 31 | 55 | 74 |
| **a25** | 24 | 41 | 104 |
| **a26** | 36 | 39 | 88 |
| **a27** | 32 | 54 | 112 |
| **a28** | 31 | 55 | 74 |
| **a29** | 30 | 54 | 94 |
| **a30** | 12 | 46 | 111 |
| **a31** | 32 | 54 | 76 |
| **a32** | 21 | 63 | 107 |
| **a33** | 21 | 63 | 107 |
| **a34** | 21 | 43 | 107 |
| **a35** | 21 | 43 | 107 |
| **a36** | 38 | 43 | 107 |
| **a37** | 14 | 60 | 71 |
| **a38** | 37 | 52 | 82 |
| **a39** | 23 | 57 | 79 |
| **a40** | 18 | 43 | 66 |
| **a41** | 23 | 42 | 93 |
| **a42** | 25 | 42 | 106 |
| **a43** | 25 | 43 | 102 |
| **a44** | 23 | 42 | 92 |
| **a45** | 22 | 42 | 92 |
| **a46** | 23 | 43 | 89 |
| **a47** | 23 | 45 | 77 |
| **a48** | 23 | 42 | 87 |
| **a49** | 23 | 42 | 83 |
| **a50** | 26 | 43 | 114 |
| **a51** | 17 | 42 | 91 |
| **a52** | 23 | 42 | 93 |
| **a53** | 37 | 53 | 72 |
| **a54** | 22 | 42 | 92 |
| **a55** | 7 | 65 | 70 |
| **a56** | 32 | 54 | 81 |
| **a57** | 13 | 49 | 101 |
| **a58** | 23 | 42 | 84 |
| **a59** | 10 | 64 | 108 |
| **a60** | 14 | 61 | 73 |
| **a61** | 20 | 43 | 78 |
| **a62** | 15 | 62 | 95 |
| **a63** | 9 | 58 | 96 |
| **a64** | 25 | 42 | 106 |
| **a65** | 13 | 47 | 100 |
| **a66** | 16 | 44 | 103 |
| **a67** | 32 | 54 | 97 |
| **a68** | 19 | 42 | 80 |
| **a69** | 23 | 43 | 78 |
| **a70** | 29 | 51 | 68 |
| **a71** | 8 | 42 | 113 |

and/or LCDR1, LCDR2 and LCDR3 as shown in any group of the following Group b1 to b71:

**Table 2**

| Group | LCDR1 | LCDR2 | LCDR3 |
|---|---|---|---|
| **b1** | 126 | 146 | 160 |
| **b2** | 126 | 146 | 160 |
| **b3** | 126 | 147 | 160 |
| **b4** | 126 | 147 | 160 |
| **b5** | 141 | 148 | 161 |
| **b6** | 133 | 149 | 162 |
| **b7** | 130 | 150 | 163 |
| **b8** | 125 | 150 | 163 |
| **b9** | 121 | 151 | 164 |
| **b10** | 144 | 152 | 165 |
| **b11** | 124 | 153 | 166 |
| **b12** | 117 | 154 | 167 |
| **b13** | 139 | 148 | 168 |
| **b14** | 128 | 147 | 160 |
| **b15** | 118 | 154 | 169 |
| **b16** | 134 | 148 | 168 |
| **b17** | 117 | 154 | 167 |
| **b18** | 126 | 146 | 160 |
| **b19** | 141 | 148 | 168 |
| **b20** | 135 | 148 | 168 |
| **b21** | 135 | 148 | 168 |
| **b22** | 141 | 148 | 170 |
| **b23** | 124 | 153 | 171 |
| **b24** | 127 | 147 | 172 |
| **b25** | 141 | 148 | 173 |
| **b26** | 124 | 153 | 174 |
| **b27** | 119 | 155 | 175 |
| **b28** | 132 | 149 | 176 |
| **b29** | 138 | 148 | 177 |
| **b30** | 118 | 154 | 178 |
| **b31** | 133 | 156 | 179 |
| **b32** | 134 | 148 | 168 |
| **b33** | 134 | 148 | 168 |
| **b34** | 134 | 148 | 168 |
| **b35** | 134 | 148 | 168 |
| **b36** | 135 | 148 | 168 |
| **b37** | 144 | 152 | 165 |
| **b38** | 133 | 149 | 180 |
| **b39** | 144 | 152 | 165 |
| **b40** | 141 | 148 | 181 |
| **b41** | 140 | 148 | 182 |
| **b42** | 141 | 148 | 183 |
| **b43** | 141 | 148 | 168 |
| **b44** | 139 | 148 | 184 |
| **b45** | 142 | 148 | 182 |
| **b46** | 140 | 148 | 182 |
| **b47** | 141 | 148 | 168 |
| **b48** | 136 | 148 | 185 |
| **b49** | 141 | 148 | 186 |
| **b50** | 143 | 155 | 187 |
| **b51** | 145 | 148 | 188 |
| **b52** | 118 | 157 | 189 |
| **b53** | 129 | 158 | 190 |
| **b54** | 141 | 148 | 183 |
| **b55** | 136 | 148 | 191 |
| **b56** | 124 | 153 | 174 |
| **b57** | 140 | 148 | 182 |
| **b58** | 137 | 148 | 168 |
| **b59** | 116 | 154 | 192 |
| **b60** | 140 | 148 | 182 |
| **b61** | 131 | 148 | 193 |
| **b62** | 123 | 159 | 194 |
| **b63** | 120 | 155 | 195 |
| **b64** | 138 | 148 | 196 |
| **b65** | 130 | 147 | 172 |
| **b66** | 141 | 148 | 197 |
| **b67** | 136 | 148 | 185 |
| **b68** | 122 | 148 | 198 |
| **b69** | 115 | 148 | 193 |
| **b70** | 118 | 155 | 199 |
| **b71** | 141 | 148 | 182 |

More preferably, the anti-OX40 antibody contains HCDR and LCDR of any one of groups c1 to c71 in Table 3.

In one or more embodiments, the FR1 of the VH of the anti-OX40 antibody can be selected from the FR1 of the VH of each antibody numbering in Table 4, FR2 of VH can be selected from FR2 of VH of each antibody numbering in Table 4, FR3 of VH can be selected from FR3 of VH of each antibody numbering in Table 4; and/or FR1 of VL can be selected from FR1 of VL of each antibody numbering in Table 4, FR2 of VL can be selected from FR2 of VL of each antibody numbering in Table 4, FR3 of VL can be selected from FR3 of VL of each antibody numbering in Table 4, FR4 of VL can be selected from FR4 of VL of each antibody numbering in Table 4.

In one or more embodiments, the FR region of the VH of the anti-OX40 antibody is selected from the FR region of any VH of SEQ ID NO: 200-270, and the FR region of VL is selected from the FR region of any VL of SEQ ID NO: 271-341.

In one or more embodiments, the amino acid sequence of the VH of the anti-OX40 antibody is as shown in any of SEQ ID NOs: 200-270, and/or the amino acid sequence of the VL is as shown in any of SEQ ID NOs: 271-341. Preferably, the amino acid sequence of VH and VL of the anti-OX40 antibody is as shown in any row of Table 4.

In some embodiments, the amino acid sequence of the heavy chain constant region of the antibody of the present description is as shown in any one of SEQ ID NO: 342-345, and/or the amino acid sequence of the light chain constant region is as shown in SEQ ID NO: 346 or 347.

In one or more embodiments, the anti-OX40 antibody, according to any embodiment of the present description, is a chimeric antibody or a complete human antibody, preferably a complete human antibody.

The present description also provides a pharmaceutical composition, which contains the anti-OX40 antibody or antigen-binding fragment thereof according to any embodiment of the present description, and a pharmaceutically acceptable excipient or carrier.

The present description also provides the use of the anti-OX40 antibody or its antigen-binding fragment according to any embodiment of the present description in the manufacture of a medicament for the treatment of T cell-related diseases. Preferably, the T cell-related disease is a T cell-related tumor or an OX40-mediated disease.

In one or more embodiments, the OX40-mediated disorders include OX40-mediated allergy, asthma, COPD, rheumatoid arthritis, psoriasis, autoimmune diseases, and inflammation-related diseases.

The present description also provides a method for treating or preventing T cell-related diseases, the method comprising administering to a patient in need a therapeutically effective amount of the anti-OX40 antibody or antigen-binding fragment thereof according to any embodiment of the present description, or a pharmaceutical composition containing the anti-OX40 antibody or antigen-binding fragment thereof according to any embodiment of the present description. Preferably, the T cell-related disease is a T cell-related tumor or an autoimmune disease.

### BRIEF DESCRIPTION OF THE DRAWINGS

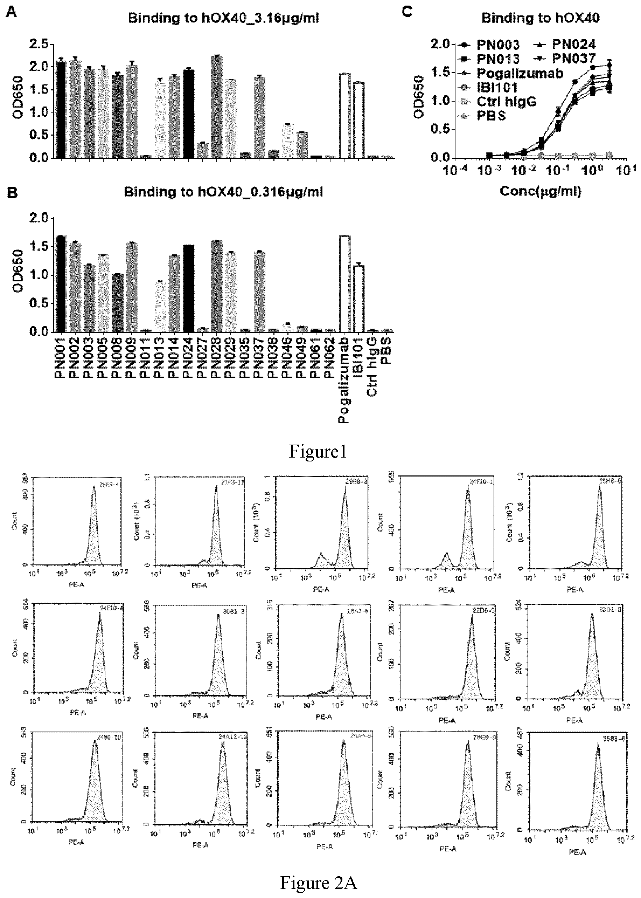

Figure 1 shows the enzyme-linked immunosorbent assay (ELISA) method to determine the binding ability of anti-human OX40 antibody derived from single-cell sequencing to human OX40 extracellular segment antigen. The results showed that for the anti-human OX40 antibodies derived from single-cell sequencing, except for a few antibodies, a certain concentration of purified single-cell sequencing antibodies all have certain binding ability to human OX40 extracellular segment antigen: (A) a concentration of 3.16µg/ml, (B) a concentration of 0.316 µg/ml, (C) gradient dilution (3.16µg/ml-1ng/ml).

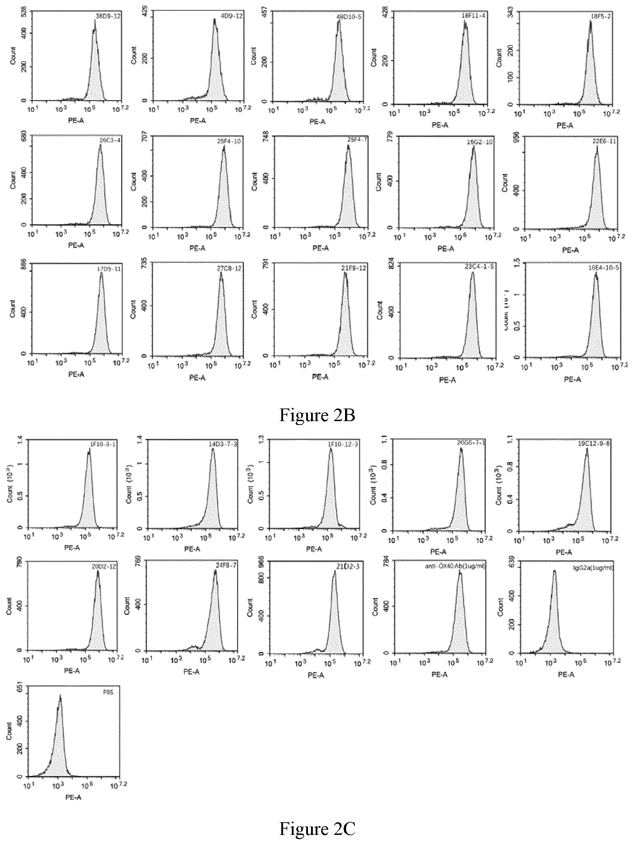

Figure 2A-C shows the results of the detection of anti-human OX40 antibody binding to the OX40 expressed on the surface of OX40 expressing cell by flow cytometry.

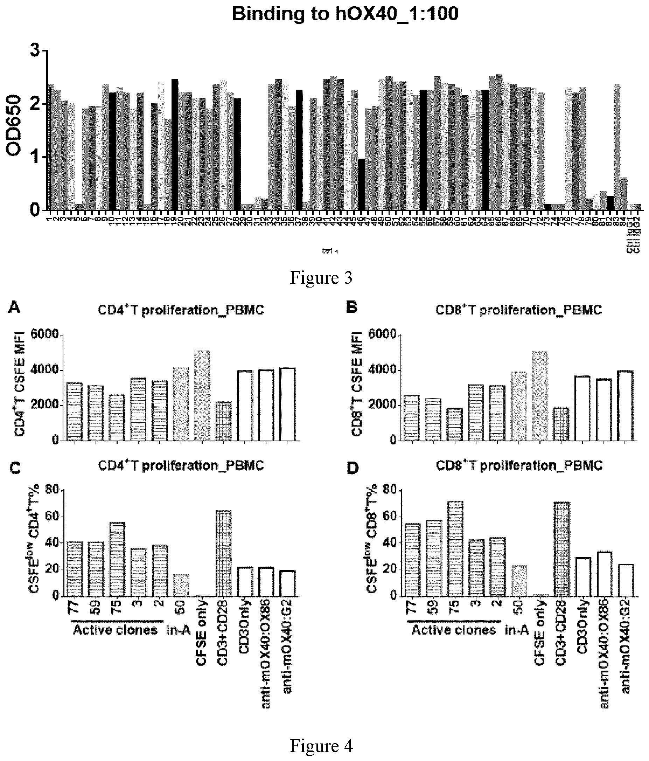

Figure 3 is the test result of antigen binding activity, showing that the anti-human OX40 antibody in the hybridoma supernatant can specifically bind human OX40 extracellular segment antigen. The binding of the anti-human OX40 antibody in the hybridoma supernatant to the human OX40 extracellular fragment antigen was analyzed by ELISA, showing the ELISA signal (A650) detected by HRP-labeled anti-mouse IgG Fc after binding of various antibodies diluted (1:100) in the supernatant to the coated human OX40.
Figure 4 shows the detection of the agonistic activity of the anti-human OX40 antibody to activate T cells and promote their proliferation in the hybridoma supernatant in human peripheral blood mononuclear cells (PBMC). The hybridoma supernatant was diluted (1:5), and the concentration of the control antibody was adjusted to 1µg/ml. The cells were cultured in the primary cell culture medium containing 0.1 µg/ml of anti-human CD3 for 72 hours, and then the mean fluorescence intensity (MFI) of CFSE of mouse CD4+ T cells was analyzed by flow cytometry. Since CFSE fluorescence intensity will be diluted proportionally during the process of cell division and proliferation, the MFI of CFSE of CD4+ T cells is negatively correlated with cell proliferation and activation, that is, negatively correlated with the activity of anti-OX40 antibodies.

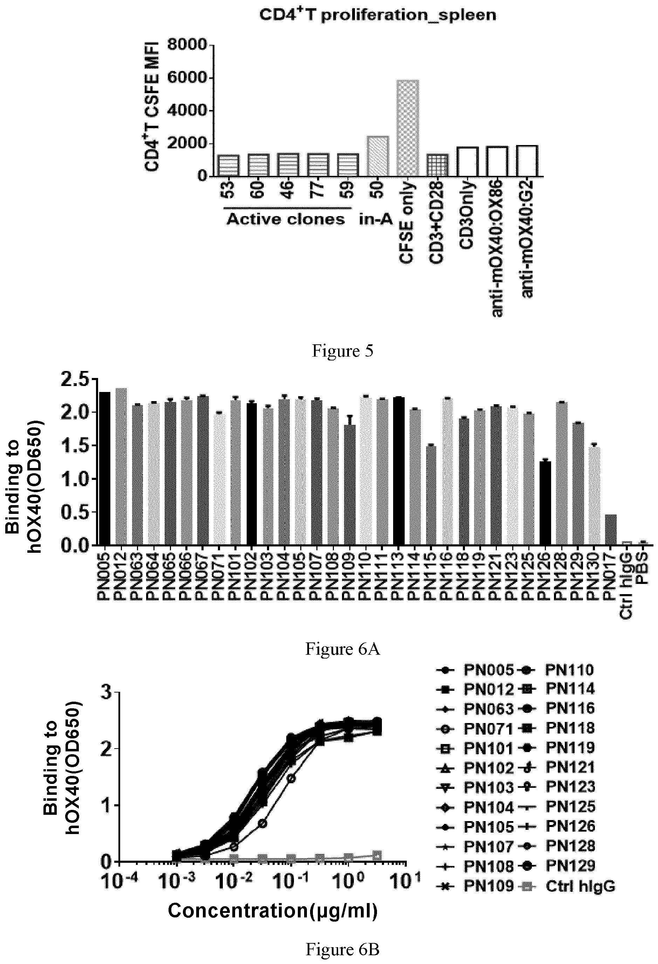

Figure 5 shows the detection of the agonistic activity of the anti-human OX40 antibody to activate T cells and promote their proliferation in the hybridoma supernatant in hOX40^{Tg} mouse spleen cells. The hybridoma supernatant was diluted (1:4), and the control antibody concentration was adjusted to 1 µg/ml. The cells were cultured in the primary cell culture medium containing 0.1 µg/ml of anti-mouse CD3 for 72 hours, and then the mean fluorescence intensity (MFI) of CFSE of (A) mouse CD4+ T cells and (B) CD8+ T cells, and the proportion of (C) CD4+ T cells and (D) CD8+ T cell with lower MFI of CFSE were analyzed by flow cytometry. Since CFSE fluorescence intensity will be diluted proportionally during the process of cell division and proliferation, the MFI of CFSE is negatively correlated with cell proliferation and activation, and the proportion of cells with lower CFSE is positively correlated with the activity of anti-OX40 antibodies.
Figure 6A-B shows the binding ability of anti-human OX40 recombinant antibody to human OX40 extracellular segment antigen determined by enzyme-linked immunosorbent assay (ELISA). The results show that, (Figure 6A) 3.16 µg/ml of anti-human OX40 recombinant antibody (PN005/012/017/063/064/065/066/067/071) derived from single cell sequencing and anti-human OX40 recombinant antibodies (PN101/102/103/104/105/107/108/109/110/111/113/114/115/116/118/119/121/12 3/125/126/128/129/130) derived from hybridoma technology all were able to bind to human OX40 extracellular fragment antigen, with Ctrl hIgG and PBS as negative controls; (Figure 6B) part of the screened antibodies were diluted in a concentration gradient (3.16 µg/ml -1 ng/ml), all of which showed the binding ability to the human OX40 extracellular segment antigen, and Ctrl hIgG was used as a negative control.

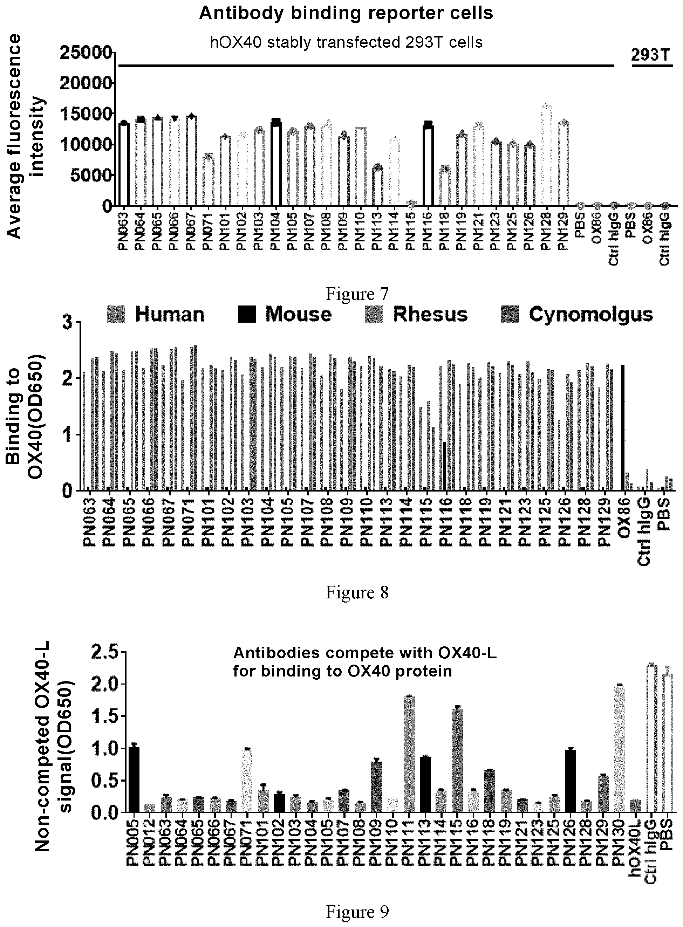

Figure 7 shows the ability of anti-human OX40 recombinant antibody to bind human OX40 on 293T cells stably transfected with hOX40 detected by flow cytometry. The results showthe signal intensity of the antibody binding to the hOX40 stably transfected 293T cell. Both recombinant antibodies PN063/064/065/066/067/071 and PN101/102/103/104/105/107/108/109/110/113/114/115/116/118/119/121/123/12 5/126/128/129 can bind OX40 on hOX40 stably transfected 293T cells, and the binding ability of PN071/113/115/118 was relatively weak. PBS, Ctrl hIgG, and OX86 (anti-mouse OX40 hIgG 1) were negative controls, and 293T was used as a negative control cell line for the reporter cells.
Figure 8 shows ELISA detection of cross-reactivity of recombinant antibodies with monkey and mouse OX40 proteins. The results showed that all the screened antibodies could bind to human and monkey (cynomolgus and macaque) OX40 proteins at the same time, and except for PN116, none of them recognized mouse OX40. PBS, Ctrl hIgG, and OX86 (anti-mouse OX40 hIgG 1) were negative controls.
Figure 9 shows the ability of recombinant antibodies to compete with human OX40 ligand (OX40-L) for its binding to OX40 protein. The results showed that when 2 µg/ml of recombinant antibody competed with 0.2 µg/ml of OX40-L-mFc for the binding to OX40 protein, PN012/063/064/065/066/067, PN101/102/103/104/105/107/108/110/114/116/119/121/123/125/128 and OX40-L-mFc have an apparent epitope competition relationship. However, PN005/071/109/111/113/115/118/126/129/130 had a weak ability to compete with OX40-L-mFc for its binding epitopes. OX40-L-His was used as a positive control, and Ctrl hIgG and PBS were used as negative controls.

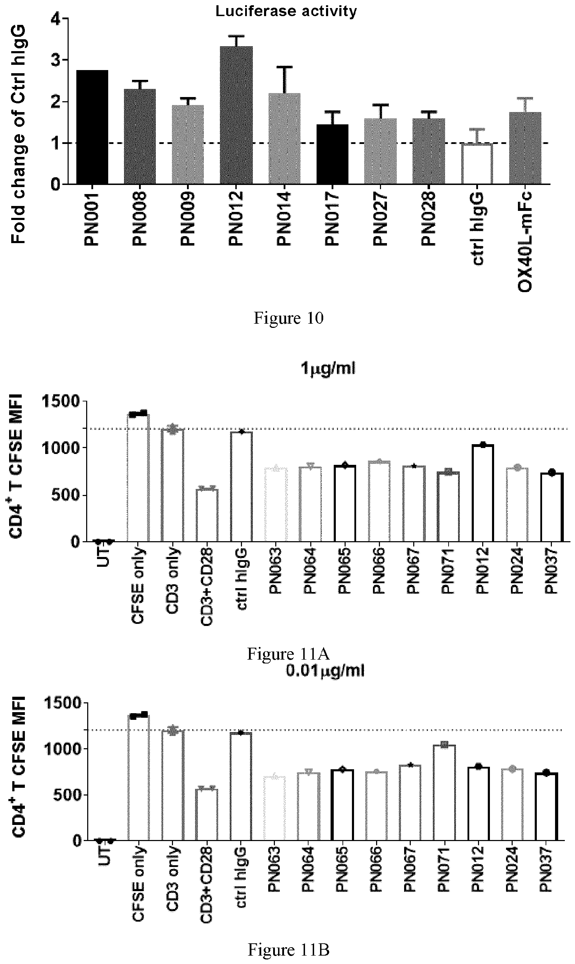

Figure 10 shows the ability of recombinant antibodies to activate OX40/NFκB reporter cells. The results showed that the 0.03 µg/ml anti-OX40 recombinant antibody clones PN001/008/009/012/014/017/027/028 have the ability to activate reporter cells compared to Ctrl hIgG. Ctrl hIgG was the negative control, and OX40-L-mFc was a positive control.

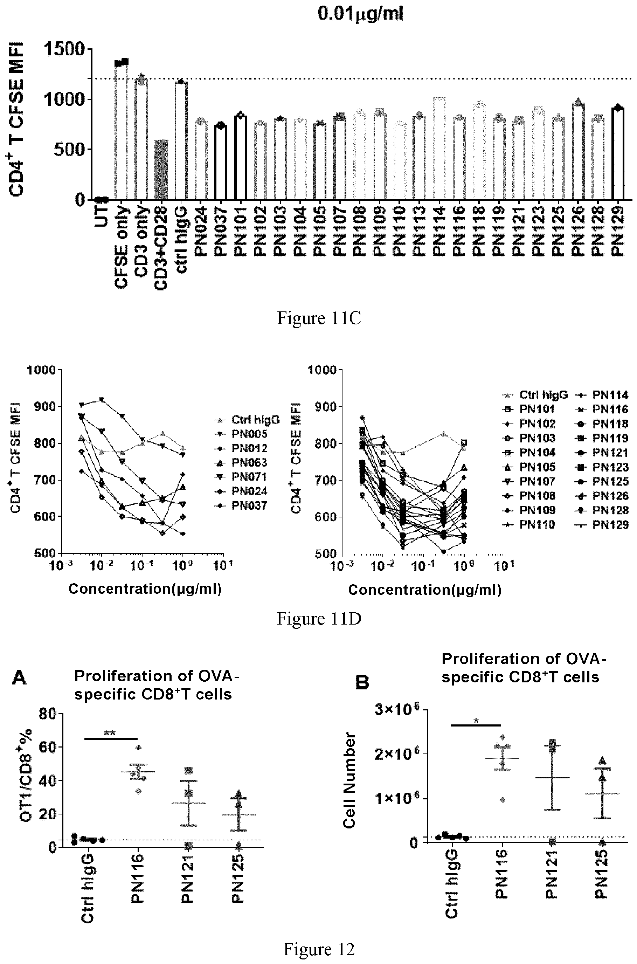

Figures 11A-D show the experimental results of recombinant antibodies activating T cells in the spleen of hFCGR^{Tg}hOX40^{Tg} mice in vitro and promoting their proliferation. The results showed that the recombinant antibodies PN012/024/037/063/063/065/066/067/071 derived from single cells have the ability to activate T cells and promote their proliferation at a high concentration (1 µg/ml) (Figure 11A); (Figure 11B) After lowering the antibody concentration (0.01 µg/ml), the single-cell-derived recombinant antibodies PN012/024/037/063/064/065/066/067/071 still showed the ability to activate T cells and promote their proliferation; (Figure 11C) At a low antibody concentration (0.01µg/ml), hybridoma-derived recombinant antibodies PN101/102/103/104/105/107/108/109/110/113/114/116/118/119/121/123/125/12 6/128/129 have the ability to activate T cells and promote their proliferation. Ctrl hIgG was the negative control of antibody, UT was the untreated group, CFSE only was the cells stained with CFSE, CD3 only was the cells stained with CFSE and stimulated with anti-mouse CD3 antibody. CD3+CD28 cells were stained with CFSE and stimulated with anti-mouse CD3 and anti-mouse CD28 antibodies (positive control). (Figure 11D) Under the stimulation of different concentrations of antibody (3.16 ng/ml -1 µg/ml), recombinant antibodies PN005/012/024/037/063/071 from single cell sequencing and PN101/102/103/104/105/107/108/109/110/114/116/118/119/121/123/125/126/12 8/129 from hybridoma technology all showed the activity of promoting T cell proliferation, and the activity was significantly higher than that of the negative control Ctrl hIgG. The fluorescence intensity of CFSE will be diluted proportionally during cell division and proliferation, so the MFI of CFSE is negatively correlated with the activation of cell proliferation, that is, negatively correlated with the activity of anti-OX40 antibody.
Fig. 12 shows that the recombinant antibody against human OX40 has the ability to activate T cell proliferation in vivo. By detecting the proliferation of OVA-specific CD8+ T cells, it can be seen that recombinant anti-human OX40 antibodies PN116/121/125/128 of 50 µg/mouse have the ability to stimulate the proliferation of OVA-specific CD8+ T cells. (A) proportion of OVA-specific CD8+(CD8+ OT1) to total CD8+ T cells, (B) total number of OVA-specific CD8+(CD8+ OT1) cells. Ctrl hIgG was used as a negative control, and OT1 cells used in this experiment were OX40 humanized and OVA-specific CD8+ cells.

### DETAILED DESCRIPTION

Unless otherwise defined, the practice of the present description will employ conventional techniques of molecular biology (including recombinant techniques), microbiology, cell biology, biochemistry and immunology, which are within the skill of the art. These techniques are well explained in the literature, such as Molecular Cloning: A Laboratory Manual, Second Edition (Sambrook et al., 1989); Oligonucleotide Synthesis (M. J. Gait ed., 1984); Animal Cell Culture (R. I. Freshney ed., 1987); Methods in Enzymology (Academic Press, Inc.) ; Current Protocols in Molecular Biology (edited by F. M. Ausubel et al., 1987 edition and its periodic updates); PCR: The Polymerase Chain Reaction (edited by Mullis et al., 1994); A Practical Guide to Molecular Cloning (Perbal Bernard V, 1988); Phage Display: A Laboratory Manual (Barbas et al., 2001).

OX40 is a cell surface receptor or OX40-containing receptor complex that binds OX40L (CD252, TNFSF4). The NCBI accession number for the amino acid sequence of human OX40 (hOX40) is (NP_003318). The OX40 protein may also include variants and fragments. The fragments include fragments that do not have all or part of the transmembrane extracellular domain, and/or the intracellular domain as well as the extracellular domain. The soluble form of hOX40 includes an extracellular domain or a fragment of the extracellular domain that retains the ability to bind BAFF and/or APRIL. "OX40" also includes post-translational modifications of the OX40 amino acid sequence. Post-translational modifications include, but are not limited to, N- and O-linked glycosylation.

OX40 is predominantly expressed on activated effector T cells (Teffs) and regulatory T cells (Tregs), as well as on NKT cells, NK cells, and neutrophils. In cancer, OX40-expressing activated T cells are found in tumor-infiltrating lymphocytes. OX40 and its ligand OX40L play a key role in the induction and maintenance of T cell responses. Enhancing anti-tumor T cell function can be useful against cancer as well as other OX40-mediated disorders, including, for example, OX40-mediated allergy, asthma, COPD, rheumatoid arthritis, psoriasis, autoimmune diseases, and inflammation-related diseases.

### Anti-QX40 antibody

This description provides antibodies that specifically bind OX40.

The term "antibody" includes monoclonal antibodies (including full-length antibodies which have an immunoglobulin Fc region), antibody compositions with polyepitopic specificity, multispecific antibodies (e.g., bispecific antibodies), diabodies, and single-chain molecules, as well as antibody fragments, e.g., Fab, F(ab')2, and Fv). The term "immunoglobulin" (Ig) is used interchangeably with "antibody" herein.

The basic four-chain antibody unit is a heterotetrameric glycoprotein composed of two identical light (L) chains and two identical heavy (H) chains. An IgM antibody consists of five basic heterotetramer units and an additional polypeptide called J chain, containing 10 antigen binding sites. While IgA antibodies comprise two to five basic four-chain units which can polymerize to form polyvalent assemblages in combination with the J chain. In the case of IgGs, the four-chain unit is generally about 150,000 daltons. Each L chain is linked to an H chain by one covalent disulfide bond, while the two H chains are linked to each other by one or more disulfide bonds depending on the H chain isotype. Each H and L chain also has regularly spaced intrachain disulfide bridges. Each H chain has a variable domain (VH) at the N-terminus, followed by three constant domains (CH) for each of the α and y chains and four CH domains for µ and ε isotypes. Each L chain has a variable domain (VL) at the N-terminus, followed by a constant domain at its other end. The VL is aligned with the VH and the CL is aligned with the first constant domain of the heavy chain (CH1). Particular amino acid residues are believed to form an interface between the light chain and heavy chain variable domains. The pairing of a VH and VL together forms a single antigen-binding site. For the structure and properties of the different classes of antibodies, see e.g., Basic and Clinical Immunology, 8th Edition, Daniel P. Sties, Abba I. Terr and Tristram G. Parsolw (eds), Appleton & Lange, Norwalk, CT, 1994, page 71 and Chapter 6. The L chain from any vertebrate species can be assigned to one of two clearly distinct types, called kappa and lambda, based on the amino acid sequences of their constant domains. Depending on the amino acid sequence of the constant domain of their heavy chains (CH), immunoglobulins can be assigned to different classes or isotypes. There are five classes of immunoglobulins: IgA, IgD, IgE, IgG and IgM, having heavy chains designated α, δ, ε, γ and µ, respectively. The γ and α classes are further divided into subclasses on the basis of relatively minor differences in the CH sequence and function, e.g., humans express the following subclasses: IgG1, IgG2A, IgG2B, IgG3, IgG4, IgA1 and IgA2.

The "variable region" or "variable domain" of an antibody refers to the amino-terminal domains of the heavy or light chain of the antibody. The variable domains of the heavy chain and light chain may be referred to as "VH" and "VL", respectively. These domains are generally the most variable parts of the antibody (relative to other antibodies of the same class) and contain the antigen binding sites.

The term "variable" refers to the fact that certain segments of the variable domains differ extensively in sequence among antibodies. The V domain mediates antigen binding and defines the specificity of a particular antibody for its specific antigen. However, the variability is not evenly distributed in the secondary sequence (amino acid sequence) across the variable domains. Instead, it is mainly in three segments called hypervariable regions (HVRs) existing in both the VH and VL, i.e., LCDR1, LCDR2 and LCDR3 in VL and HCDR1, HCDR2 and HCDR3 in VH. The highly conserved portions of variable domains are called the framework regions (FR). The variable domains of native heavy and light chains, each comprise four FR regions (FR1, FR2, FR3 and FR4), largely adopting a beta-sheet configuration, connected by three HVRs, which form connecting loops, and in some cases forming part of, the beta-sheet structure. The HVRs in each chain are held together in close proximity by the FR regions and, with the HVRs from the other chain, contribute to the formation of the antigen binding site of antibodies (see Kabat et al, Sequences of Immunological Interest, Fifth Edition, National Institute of Health, Bethesda, MD (1991)). Typically, the structure of the light chain variable region is FR1-LCDR1-FR2-LCDR2-FR3-LCDR3-FR4, and the structure of the heavy chain variable region is FR1-HCDR1-FR2-HCDR2-FR3-HCDR3-FR4. The constant domains are not involved directly in the binding of antibody to an antigen, but exhibit various effector functions, such as engaging the antibody in antibody-dependent cellular toxicity.

In the present description, some antibodies are agonistic antibodies, and some antibodies are non-agonistic antibodies. Preferably, the CDRs of the agonistic antibody of the present description can be combined arbitrarily, so as to constitute the CDR group of the agonistic antibody or be used to form the variable region of the agonistic antibody; The CDRs of the non-agonist antibody of the present description can be combined arbitrarily to form an effective CDR group, thereby constituting the CDR group of the non-agonist antibody or used to form the variable region of the non-agonist antibody. Herein, "agonistic antibody variable region" refers to a variable region that has agonistic activity against an antigen in the form of an intact antibody, such as an OX40 agonistic antibody variable region or an agonistic OX40 antibody variable region; "Non-agonistic antibody variable region" refers to an antibody variable region that does not have any agonistic activity in the form of an intact antibody, such as an OX40 non-agonistic antibody variable region or a non-agonistic OX40 antibody variable region.

The term "monoclonal antibody" as used herein refers to an antibody obtained from a population of substantially homogeneous antibodies, i.e., the individual antibodies comprising the population are identical except for potential naturally occurring mutations and/or post-translation modifications (e.g., isomerization, amination) that may be present minorly. Monoclonal antibodies are highly specific, being directed against a unique epitope. In contrast to polyclonal antibodies which typically contain different antibodies directed against various epitopes, each monoclonal antibody is directed against a unique epitope on the antigen. In addition to their specificity, the monoclonal antibodies are advantageous in that they are produced by the hybridoma culture, devoid of the contamination by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present description may be made by a variety of techniques, including, for example, the hybridoma (e.g., Kohler and Milstein., Nature, 256:495-97 (1975); Hongo et al, Hybridoma, 14 (3): 253-260 (1995), Harlow et al, Antibodies: A Laboratory Manual, (Cold Spring Harbor Laboratory Press, 2nd ed. 1988); Hammerling et al, in: Monoclonal Antibodies and T-CeIl Hybridomas 563-681 (Elsevier, N. Y, 1981)), DNA recombination (see, e.g., U.S. Patent No. 4,816,567), phage-display (see, e.g., Clackson et al, Nature, 352: 624-628 (1991); Marks et al, J. MoI Biol. 222: 581-597 (1992); Sidhu et al, J. MoI Biol. 338(2): 299-310 (2004); Lee et al, J. MoI Biol. 340(5): 1073-1093 (2004); Fellouse, Proc. Natl. Acad. ScL USA 101(34): 12467-12472 (2004); and Lee et al, J. Immunol. Methods 284(1-2): 119-132 (2004), and technologies for producing human or humanlike antibodies in animals that have parts or all of the human immunoglobulin loci or genes encoding human immunoglobulin sequences (see, e.g., WO 1998/24893; WO 1996/34096; WO 1996/33735; WO 1991/10741; Jakobovits et al, Proc. Natl. Acad. ScL USA 90: 2551 (1993); Jakobovits et al, Nature 362: 255-258 (1993); Bruggemann et al, Year in Immunol. 7:33 (1993); U.S. Patent Nos. 5,545,807; 5,545,806; 5,569,825; 5,625,126; 5,633,425; and 5,661,016; Marks et al, Bio/Technology 10: 779-783 (1992); Lonberg et al, Nature 368: 856-859 (1994); Morrison, Nature 368: 812-813 (1994); Fishwild et al, Nature Biotechnol 14: 845-851 (1996); Neuberger, Nature Biotechnol. 14: 826 (1996); and Lonberg and Huszar, Intern. Rev. Immunol. 13: 65-93 (1995), Single-cell sequencing method (Nat Biotechnol. 2013 Feb;31(2):166-9.)

The terms "full-length antibody," "intact antibody" or "whole antibody" are used interchangeably to refer to an antibody in its substantially intact form, as opposed to an antibody fragment. Specifically whole antibodies include those with heavy and light chains including an Fc region. The constant domains may be native sequence constant domains (e.g., human native sequence constant domains) or amino acid sequence variants thereof. In some cases, the intact antibody may have one or more effector functions.

An "antibody fragment" comprises a portion of an intact antibody, preferably the antigen binding and/or the variable region of the intact antibody. Antibody fragments are preferably antigen-binding fragments of antibodies. Examples of antibody fragments include Fab, Fab', F(ab')2 and Fv fragments; diabodies; linear antibodies (see U.S. Patent 5,641,870, Example 2; Zapata et al, Protein Eng., 8(10): 1057-1062, 1995); single-chain antibody molecules; scFv-Fc fragments; multispecific antibodies formed from antibody fragments; and any fragment that should be able to increase half-life by chemical modification or by incorporation into liposomes. Papain digestion of antibodies produced two identical antigen-binding fragments, called "Fab" fragments, and a residual "Fc" fragment, a designation reflecting the ability to crystallize readily. The Fab fragment consists of an entire L chain along with the variable region domain of the H chain (VH), and the first constant domain of one heavy chain (CH1). Each Fab fragment is monovalent with respect to antigen binding, i.e., it has a single antigen-binding site. Pepsin treatment of an antibody yields a single large F(ab')2 fragment which roughly corresponds to two disulfide linked Fab fragments having different antigen-binding activity and is still capable of cross-linking antigen. Fab' fragments differ from Fab fragments by having a few additional residues at the carboxy terminus of the CH1 domain including one or more cysteines from the antibody hinge region. F(ab')2 antibody fragments originally were produced as pairs of Fab' fragments which have hinge cysteines between them. Other chemical couplings of antibody fragments are also known. The Fc fragment comprises the carboxy-terminal portions of both H chains held together by disulfides. The effector functions of antibodies are determined by sequences in the Fc region, the region which is also recognized by Fc receptors (FcR) found on certain types of cells.

"Fv" is the minimum antibody fragment which contains a complete antigen-recognition and -binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three HVRs specific for an antigen) can recognize and bind antigen, although at a lower affinity than the entire binding site.

"Single-chain Fv " also abbreviated as "sFv " or "scFv " are antibody fragments that comprise the VH and VL antibody domains connected into a single polypeptide chain. Preferably, the sFv polypeptide further comprises a polypeptide linker between the VH and VL domains which enables the sFv to form the desired structure for antigen binding. For a review of the sFv, refer to/see The Pharmacology of Monoclonal Antibodies, vol. 113, Rosenburg and Moore eds., Springer- Verlag, New York, pp. 269-315 (1994).

"Chemical modification" of the fragments includes the addition of poly(alkylene) glycols such as polyethylene glycol ("PEGylated, PEGylated"), including PEGylated fragments of Fv, scFv, Fab, F(ab')2 and Fab ', namely Fv-PEG, scFv-PEG, Fab-PEG, F(ab')2-PEG and Fab'-PEG. Such fragments have EGFR binding activity.

Preferably, the antibody fragment, especially the antigen-binding fragment, consists of or comprises a partial sequence of the variable heavy or light chain of the antibody from which it is derived, said partial sequence being sufficient to retain the same binding specificity and sufficient affinity as the antibody from which it is derived, for OX40, the affinity of the antibody fragment are preferably equal to at least 1/100, and more preferably equal to at least 1/10, of the affinity of the antibody from which it is derived. Such antibody fragments will contain a minimum of 5 amino acids, preferably 10, 15, 25, 50 and 100 contiguous amino acids of the antibody sequence from which they are derived.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is(are) identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al, Proc. Natl. Acad. ScL USA, 81:6851-6855 (1984)).

"Humanized" forms of non-human (e.g., murine) antibodies are chimeric antibodies that contain minimal sequence derived from non-human immunoglobulin. Thus, a "humanized antibody" generally refers to a non-human antibody in which the variable domain framework regions are exchanged with sequences found in human antibodies. Typically in a humanized antibody, the entire antibody (except the CDRs) is encoded by a polynucleotide of human origin or is identical to such antibody (except for the CDRs). CDRs (some or all of which are encoded by nucleic acids derived from non-human organisms) are grafted into the β-sheet backbone of the variable regions of human antibodies to generate antibodies, the specificity of which is determined by the grafted CDRs. The production of such antibodies is described, for example, in WO92/11018; Jones, 1986, Nature, 321:522-525; Verhoeyen et al., 1988, Science, 239:1534-1536. Humanized antibodies can also be generated using mice with genetically engineered immune systems (see Roque et al., 2004, Biotechnol. Prog., 20:639-654).

A "human antibody" is an antibody that possesses an amino-acid sequence corresponding to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art, including phage-display libraries. Such techniques are described in Hoogenboom and Winter, J. Molecular Biology, 227:381 (1991); Marks et al., J. Molecular Biology, 222:581 (1991). Available methods for preparing human monoclonal antibodies are described in Cole et al., Monoclonal Antibodies and Cancer Therapy, Alan R. Liss, p. 77 (1985); Boerner et al., J. Immunol., 147(1): 86-95 (1991)). See also van Dijk and van de Winkel, Modern Pharmacy Reviews, 5:368-74 (2001). Human antibodies can be prepared by administering the antigen to a transgenic animal that has been modified to produce such antibodies in response to antigenic challenge, but whose endogenous loci have been disabled, e.g., immunized xenomice (see, e.g., U.S. Pat. Nos. 6,075,181 and 6,150,584 regarding XENOMOUSE^{™} technology). See also, for example, Li et al, Proc. Natl. Acad. Sci. USA, 103:3557-3562 (2006) regarding human antibodies generated via a human B-cell hybridoma technology.

OX40 antibodies of this description may also be minibodies. Minibodies are minimal antibody-like proteins comprising scFv linked to the CH3 domain (Hu et al., 1996, Cancer Res., 56:3055-3061). Anti-OX40 antibodies of the description may also be domain antibodies, see eg. US 6,248,516. Domain antibodies (dAbs) are functional binding domains of antibodies, corresponding to the variable regions of the heavy (VH) or light (VL) chains of human antibody dABs, with a molecular weight of about 13 kDa or a size less than 1/10 of an intact antibody. dABs are well expressed in a variety of hosts including bacterial, yeast and mammalian cell systems. Additionally, the dAbs are highly stable and remain active even after being subjected to harsh conditions, such as freeze-drying or thermal denaturation. See references eg. US 6,291,158; US 6,582,915; US 6,593,081; US 6,172,197; US 2004/0110941; EP 0368684; US 6,696,245; WO04/058821; WO04/003019; and WO03/002609.

HCDR1 of the anti-OX40 antibody of the present description may contain X₁X₂X₃X₄X₅X₆X₇X₈X₉X₁₀ (SEQ ID NO:1), wherein X₁ is G or S; X₂ is A, D, F, G, V or Y; X₃ is S, T or I; X₄ is I, L or F; X₅ is I, S, D, N, T or A; X₆ is N, S, T, D, R or G; X₇ is N, S, T, Y, A, G or F; X₈ is N, Y, A, G, W, S, I, K, F, T or D; X₉ is W, V or none; X₁₀ is N, G or none. The amino acid sequence of exemplary HCDR1 is shown in any of SEQ ID NO: 7-38. In one or more embodiments, X₁ is G or S; X₂ is D, F, G or Y; X₃ is S or T; X₄ is I, L or F; X₅ is S, D or T; X₆ is N, S, T or D; X₇ is S, T or Y; X₈ is N, Y, A, G or I; X₉ is W, V or none; X₁₀ is N or none. Preferably, the amino acid sequence of HCDR1 is shown in any of SEQ ID NO: 9, 10, 12, 13, 19, 21, 23, 33, 34, 38.

HCDR2 of the anti-OX40 antibody of the present description may contain X₁X₂X₃X₄X₅X₆X₇X₈X₉X₁₀ (SEQ ID NO:2), wherein X₁ is M or I; X₂ is N, Y, S, K, H or F; X₃ is P, W, Q, H, S, G, A or R; X₄ is N, S, G, Y, K or D; X₅ is S, D, G, N, A or T; X₆ is T, N, S, D or G; X₇ is N, K, T, S, D, Y, H, G, A or E; X₈ is T, I, D, G or none; X₉ is T or none; X₁₀ is T, I or none. In one or more embodiments, X₁ is I; X₂ is N, Y, S, H or F; X₃ is W, H, S or A; X₄ is N, S, G or Y; X₅ is S, D, G or N; X₆ is T, N, S, D or G; X₇ is N, K, T, S or D; X₈ is T, I, K or none; X₉ and X₁₀ are none. An exemplary amino acid sequence of HCDR2 is shown in any one of SEQ ID NO: 39-65. Preferably, the amino acid sequence of HCDR2 is as shown in any one of SEQ ID NO: 42, 43, 46, 47, 50, 58, 59, 63, 64.

HCDR3 of the anti-OX40 antibody of the description can contain CX₁X₂X₃X₄X₅X₆X₇X₈X₉X₁₀X₁₁X₁₂X₁₃X₁₄X₁₅X₁₆X₁₇X₁₈ (SEQ ID NO: 3), wherein X₁ is V, T, S or A, X₂ is R, K, I, G, A, T, S, H, Q, N or E, X₃ is S, D, W, G, Y, A, E, R, L, V, T, M or K, X₄ is G, Y, F, V, L, A, P, D, S, W, R, Q, E, N or K, X₅ is D, Y, G, S, R, F, T, V, A, P, L, I, E, W or N, X₆ is W, G, E, D, S, R P, Y, N, L, A, T, I or V, X₇ is H, S, L, A, T, Y, G, F, W, V, E, D or M, X₈ is C, G, L, F, Y, S, W, D, M, T, N, R, H, V or P, X₉ is F, S, D, Y, W, G, T, E, P, L, R, N or none, X₁₀ is D, Y, F, I, N, W, V, P, E, L, G, S, Q or none, X₁₁ is Y, G, D, W, F, A, I, Q, S, N or none, X₁₂ is W, D, Y, F, N, G, R, P or none, X₁₃ is I, W, Y, D, S F, G, L or none, X₁₄ is Q, W, I, Y, F, D, V, H or none, X₁₅ is H, W, M, D, Y, F or none, X₁₆ is W, D, S, Y or none, X₁₇ is V, W, Y or none, X₁₈ is W or none. In one or more embodiments, X₁ is V or A, X₂ is R, K, S or Q, X₃ is S, D, G, Y, A or E, X₄ is G, Y, D, S, W or N, X₅ is Y, S, F, T, A, L, I or W, X₆ is G, D, P, Y, N, L or T, X₇ is S, L, T, Y, F, W, E or D, X₈ is G, F, Y, S, W, M, T or H, X₉ is F, S, D, Y, E, L or none, X₁₀ is D, Y, V, L, G or none, X₁₁ is Y, G, W, F, Q, N or none, and X₁₂-X₁₈ is none. The amino acid sequence of LCDR1 is shown in any of SEQ ID NO: 66-114. Preferably, the amino acid sequence of HCDR3 is shown in any of SEQ ID NO: 67, 78, 79, 80, 86, 93, 96, 99, 100, 107, 108.

LCDR1 of the anti-OX40 antibody of the description can contain X₁X₂X₃X₄X₅X₆X₇X₈X₉X₁₀X₁₁X₁₂ (SEQ ID NO: 4), wherein X₁ is H or Q, X₂ is G, D, S or T, X₃ is S, I, L, T or V, X₄ is S, N, R, W, L, V or F, X₅ is T, D, N, S, H, Y or G, X₆ is Y, D, W, S, R, N, G or T, X₇ is N, D, S, Y or N, X₈ is G, N or N None, X₉ is Y, N, D, H, S or none, X₁₀ is N, T, R, K or none, X₁₁ is Y, F, S, N or none, X₁₂ is Y or none. In one or more embodiments, X₁ is H or Q, X₂ is G, D or S, X₃ is S, I, L or V, X₄ is S, N, W, L or F, X₅ is T, N, S, H, Y or G, X₆ is Y, D, S, R or N, X₇ is N, D, Y or none, X₈ is G or none, X₉ is Y, N, D or none, X₁₀ is N, T or none, X₁₁ is Y or none, X₁₂ is none. The amino acid sequence of LCDR1 is shown in any of SEQ ID NO: 115-145. Preferably, the amino acid sequence of LCDR1 is shown in any of SEQ ID NO: 115, 116, 120, 122, 124, 126, 130, 134, 135, 140, 141.

LCDR2 of the anti-OX40 antibody of the description can contain X₁X₂X₃ (SEQ ID NO: 5), wherein X₁ is T, K, G, W, E, M, L, D, A or Q; X₂ is V, A, T or G; X₃ is S or A. In one or more embodiments, X₁ is K, G, L, D or A; X₂ is V, A or G; X₃ is S. The amino acid sequence of LCDR2 is shown in any of SEQ ID NO: 146-159. Preferably, the amino acid sequence of LCDR2 is shown in any of SEQ ID NO: 147, 148, 153, 154, 155.

The LCDR3 of the anti-OX40 antibody of the description can contain CX₁QX₂X₃X₄X₅X₆X₇X₈X₉X₁₀X₁₁ (SEQ ID NO: 6), wherein X₁ is M, Q, L or H, X₂ is G, Y, A, V, H, T, F or S, X₃ is T, G, Y, N, L, D or S, X₄ is H, S, T, Q, D, N, G, I or R, X₅ is W, S, T, Y, L, I or N, X₆ is P, S, M, I, W or T, X₇ is W, F, L, P, I, Y, R or T, X₈ is T, L, A, F or W, X₉ is F, L, T or none, X₁₀ is T, F or none, X₁₁ is F or none. In one or more embodiments, X₁ is M, Q or L, X₂ is G, Y, A or S, X₃ is T, G, Y, N, L or D, X₄ is H, S, Q or N, X₅ is W, S, T, Y, L, I or N, X₆ is P or I, X₇ is W, F, L, I, Y, R or T, X₈ is T or F, X₉ is F or N, X₁₀ and X₁₁ are none. The amino acid sequence of LCDR3 is shown in any of SEQ ID NO: 160-199. Preferably, the amino acid sequence of LCDR3 is shown in any of SEQ ID NO: 160, 161, 166, 168, 172, 182, 192, 193, 195, 198.

In some embodiments, the anti-OX40 antibody of the description contains HCDR1 as shown in SEQ ID NO: 1, HCDR2 as shown in SEQ ID NO: 2, HCDR3 as shown in SEQ ID NO: 3, and/or LCDR1 as shown in SEQ ID NO: 4, LCDR2 as shown in SEQ ID NO: 5, and LCDR3 as shown in SEQ ID NO: 6. Preferably, the anti-OX40 antibody of the present description contains HCDR1 as shown in any of SEQ ID NO: 7-38, HCDR2 as shown in any of SEQ ID NO: 39-65, HCDR3 as shown in any of SEQ ID NO: 66-114, and/or LCDR1 as shown in any of SEQ ID NO: 115-145, LCDR2 as shown in any of SEQ ID NO: 146-159 and LCDR3 as shown in any of SEQ ID NO: 160-199.

More preferably, the anti-OX40 antibody of the description contains HCDR1 as shown in any of SEQ ID NO: 9, 10, 12, 13, 19, 21, 23, 33, 34, 38, HCDR2 as shown in any of SEQ ID NO: 42, 43, 46, 47, 50, 58, 59, 63, 64, and HCDR3 as shown in any of SEQ ID NO: 67, 78, 79, 80, 86, 93, 96, 99, 100, 107, 108, And/or LCDR1 as shown in SEQ ID NO: 115, 116, 120, 122, 124, 126, 130, 134, 135, 140, 141, LCDR2 as shown in SEQ ID NO: 147, 148, 153, 154, 155 and LCDR3 as shown in SEQ ID NO: 160, 161, 166, 168, 172, 182, 192, 193, 195, 198.

Further preferably, the anti-OX40 antibody of the present description contains HCDR1, HCDR2 and HCDR3 shown in any of the following groups a1 to a71:

| Group | HCDR1 | HCDR2 | HCDR3 |
|---|---|---|---|
| **a1** | 23 | 57 | 79 |
| **a2** | 23 | 57 | 79 |
| **a3** | 23 | 43 | 79 |
| **a4** | 23 | 48 | 79 |
| **a5** | 34 | 50 | 99 |
| **a6** | 35 | 59 | 110 |
| **a7** | 27 | 59 | 69 |
| **a8** | 28 | 59 | 69 |
| **a9** | 32 | 56 | 105 |
| **a10** | 23 | 42 | 85 |
| **a11** | 12 | 46 | 67 |
| **a12** | 14 | 60 | 71 |
| **a13** | 34 | 52 | 98 |
| **a14** | 11 | 40 | 75 |
| **a15** | 23 | 48 | 79 |
| **a16** | 21 | 63 | 107 |
| **a17** | 23 | 57 | 79 |
| **a18** | 23 | 57 | 109 |
| **a19** | 34 | 50 | 99 |
| **a20** | 33 | 59 | 86 |
| **a21** | 38 | 43 | 107 |
| **a22** | 32 | 54 | 90 |
| **a23** | 33 | 59 | 86 |
| **a24** | 31 | 55 | 74 |
| **a25** | 24 | 41 | 104 |
| **a26** | 36 | 39 | 88 |
| **a27** | 32 | 54 | 112 |
| **a28** | 31 | 55 | 74 |
| **a29** | 30 | 54 | 94 |
| **a30** | 12 | 46 | 111 |
| **a31** | 32 | 54 | 76 |
| **a32** | 21 | 63 | 107 |
| **a33** | 21 | 63 | 107 |
| **a34** | 21 | 43 | 107 |
| **a35** | 21 | 43 | 107 |
| **a36** | 38 | 43 | 107 |
| **a37** | 14 | 60 | 71 |
| **a38** | 37 | 52 | 82 |
| **a39** | 23 | 57 | 79 |
| **a40** | 18 | 43 | 66 |
| **a41** | 23 | 42 | 93 |
| **a42** | 25 | 42 | 106 |
| **a43** | 25 | 43 | 102 |
| **a44** | 23 | 42 | 92 |
| **a45** | 22 | 42 | 92 |
| **a46** | 23 | 43 | 89 |
| **a47** | 23 | 45 | 77 |
| **a48** | 23 | 42 | 87 |
| **a49** | 23 | 42 | 83 |
| **a50** | 26 | 43 | 114 |
| **a51** | 17 | 42 | 91 |
| **a52** | 23 | 42 | 93 |
| **a53** | 37 | 53 | 72 |
| **a54** | 22 | 42 | 92 |
| **a55** | 7 | 65 | 70 |
| **a56** | 32 | 54 | 81 |
| **a57** | 13 | 49 | 101 |
| **a58** | 23 | 42 | 84 |
| **a59** | 10 | 64 | 108 |
| **a60** | 14 | 61 | 73 |
| **a61** | 20 | 43 | 78 |
| **a62** | 15 | 62 | 95 |
| **a63** | 9 | 58 | 96 |
| **a64** | 25 | 42 | 106 |
| **a65** | 13 | 47 | 100 |
| **a66** | 16 | 44 | 103 |
| **a67** | 32 | 54 | 97 |
| **a68** | 19 | 42 | 80 |
| **a69** | 23 | 43 | 78 |
| **a70** | 29 | 51 | 68 |
| **a71** | 8 | 42 | 113 |

Preferably contain HCDR1, HCDR2 and HCDR3 selected from any of the following groups: a3, a5, a11, a16, a19, a20, a21, a41, a59, a63, a65, a68, a69; and/or LCDR1, LCDR2 and LCDR3 shown in any of the following groups b1 to b71:

| Group | LCDR1 | LCDR2 | LCDR3 |
|---|---|---|---|
| **b1** | 126 | 146 | 160 |
| **b2** | 126 | 146 | 160 |
| **b3** | 126 | 147 | 160 |
| **b4** | 126 | 147 | 160 |
| **b5** | 141 | 148 | 161 |
| **b6** | 133 | 149 | 162 |
| **b7** | 130 | 150 | 163 |
| **b8** | 125 | 150 | 163 |
| **b9** | 121 | 151 | 164 |
| **b10** | 144 | 152 | 165 |
| **b11** | 124 | 153 | 166 |
| **b12** | 117 | 154 | 167 |
| **b13** | 139 | 148 | 168 |
| **b14** | 128 | 147 | 160 |
| **b15** | 118 | 154 | 169 |
| **b16** | 134 | 148 | 168 |
| **b17** | 117 | 154 | 167 |
| **b18** | 126 | 146 | 160 |
| **b19** | 141 | 148 | 168 |
| **b20** | 135 | 148 | 168 |
| **b21** | 135 | 148 | 168 |
| **b22** | 141 | 148 | 170 |
| **b23** | 124 | 153 | 171 |
| **b24** | 127 | 147 | 172 |
| **b25** | 141 | 148 | 173 |
| **b26** | 124 | 153 | 174 |
| **b27** | 119 | 155 | 175 |
| **b28** | 132 | 149 | 176 |
| **b29** | 138 | 148 | 177 |
| **b30** | 118 | 154 | 178 |
| **b31** | 133 | 156 | 179 |
| **b32** | 134 | 148 | 168 |
| **b33** | 134 | 148 | 168 |
| **b34** | 134 | 148 | 168 |
| **b35** | 134 | 148 | 168 |
| **b36** | 135 | 148 | 168 |
| **b37** | 144 | 152 | 165 |
| **b38** | 133 | 149 | 180 |
| **b39** | 144 | 152 | 165 |
| **b40** | 141 | 148 | 181 |
| **b41** | 140 | 148 | 182 |
| **b42** | 141 | 148 | 183 |
| **b43** | 141 | 148 | 168 |
| **b44** | 139 | 148 | 184 |
| **b45** | 142 | 148 | 182 |
| **b46** | 140 | 148 | 182 |
| **b47** | 141 | 148 | 168 |
| **b48** | 136 | 148 | 185 |
| **b49** | 141 | 148 | 186 |
| **b50** | 143 | 155 | 187 |
| **b51** | 145 | 148 | 188 |
| **b52** | 118 | 157 | 189 |
| **b53** | 129 | 158 | 190 |
| **b54** | 141 | 148 | 183 |
| **b55** | 136 | 148 | 191 |
| **b56** | 124 | 153 | 174 |
| **b57** | 140 | 148 | 182 |
| **b58** | 137 | 148 | 168 |
| **b59** | 116 | 154 | 192 |
| **b60** | 140 | 148 | 182 |
| **b61** | 131 | 148 | 193 |
| **b62** | 123 | 159 | 194 |
| **b63** | 120 | 155 | 195 |
| **b64** | 138 | 148 | 196 |
| **b65** | 130 | 147 | 172 |
| **b66** | 141 | 148 | 197 |
| **b67** | 136 | 148 | 185 |
| **b68** | 122 | 148 | 198 |
| **b69** | 115 | 148 | 193 |
| **b70** | 118 | 155 | 199 |
| **b71** | 141 | 148 | 182 |

preferably, LCDR1, LCDR2 and LCDR3 are selected from any of the following groups: b3, b5, b11, b16, b19, b20, b21, b41, b59, b63, b65, b68 and b69.

More preferably, the anti-OX40 antibody of the description contains HCDR and LCDR of any group from group c1 to group c71 in Table 3:

**Table 3**

| Group | HCDR1 | HCDR2 | HCDR3 | LCDR1 | LCDR2 | LCDR3 | Recombinant antibody number | Name of corresponding hybridoma antibody (if any) |
|---|---|---|---|---|---|---|---|---|
| **c1** | 23 | 57 | 79 | 126 | 146 | 160 | PN001 | |
| **c2** | 23 | 57 | 79 | 126 | 146 | 160 | PN002 | |
| **c3** | **23** | **43** | **79** | **126** | **147** | **160** | **PN003** | |
| **c4** | 23 | 48 | 79 | 126 | 147 | 160 | PN004 | |
| **c5** | **34** | **50** | **99** | **141** | **148** | **161** | **PN005** | |
| **c6** | 35 | 59 | 110 | 133 | 149 | 162 | PN006 | |
| **c7** | 27 | 59 | 69 | 130 | 150 | 163 | PN008 | |
| **c8** | 28 | 59 | 69 | 125 | 150 | 163 | PN009 | |
| **c9** | 32 | 56 | 105 | 121 | 151 | 164 | PN011 | |
| **c10** | 23 | 42 | 85 | 144 | 152 | 165 | PN012 | |
| **c11** | **12** | **46** | **67** | **124** | **153** | **166** | **PN013** | |
| **c12** | 14 | 60 | 71 | 117 | 154 | 167 | PN014 | |
| **c13** | 34 | 52 | 98 | 139 | 148 | 168 | PN017 | |
| **c14** | 11 | 40 | 75 | 128 | 147 | 160 | PN019 | |
| **c15** | 23 | 48 | 79 | 118 | 154 | 169 | PN021 | |
| **c16** | **21** | **63** | **107** | **134** | **148** | **168** | **PN024** | |
| **c17** | 23 | 57 | 79 | 117 | 154 | 167 | PN027 | |
| **c18** | 23 | 57 | 109 | 126 | 146 | 160 | PN028 | |
| **c19** | **34** | **50** | **99** | **141** | **148** | **168** | **PN029** | |
| **c20** | **33** | **59** | **86** | **135** | **148** | **168** | **PN035** | |
| **c21** | **38** | **43** | **107** | **135** | **148** | **168** | **PN037** | |
| **c22** | 32 | 54 | 90 | 141 | 148 | 170 | PN038 | |
| **c23** | 33 | 59 | 86 | 124 | 153 | 171 | PN043 | |
| **c24** | 31 | 55 | 74 | 127 | 147 | 172 | PN045 | |
| **c25** | 24 | 41 | 104 | 141 | 148 | 173 | PN046 | |
| **c26** | 36 | 39 | 88 | 124 | 153 | 174 | PN049 | |
| **c27** | 32 | 54 | 112 | 119 | 155 | 175 | PN051 | |
| **c28** | 31 | 55 | 74 | 132 | 149 | 176 | PN053 | |
| **c29** | 30 | 54 | 94 | 138 | 148 | 177 | PN059 | |
| **c30** | 12 | 46 | 111 | 118 | 154 | 178 | PN061 | |
| **c31** | 32 | 54 | 76 | 133 | 156 | 179 | PN062 | |
| **c32** | 21 | 63 | 107 | 134 | 148 | 168 | PN063 | |
| **c33** | 21 | 63 | 107 | 134 | 148 | 168 | PN064 | |
| **c34** | 21 | 43 | 107 | 134 | 148 | 168 | PN065 | |
| **c35** | 21 | 43 | 107 | 134 | 148 | 168 | PN066 | |
| **c36** | 38 | 43 | 107 | 135 | 148 | 168 | PN067 | |
| **c37** | 14 | 60 | 71 | 144 | 152 | 165 | PN068 | |
| **c38** | 37 | 52 | 82 | 133 | 149 | 180 | PN069 | |
| **c39** | 23 | 57 | 79 | 144 | 152 | 165 | PN070 | |
| **c40** | 18 | 43 | 66 | 141 | 148 | 181 | PN071 | |
| **c41** | **23** | **42** | **93** | **140** | **148** | **182** | **PN101** | **16E4-10-5** |
| **c42** | 25 | 42 | 106 | 141 | 148 | 183 | PN102 | 24F8-7 |
| **c43** | 25 | 43 | 102 | 141 | 148 | 168 | PN103 | 21F9-12 |
| **c44** | 23 | 42 | 92 | 139 | 148 | 184 | PN104 | 23C4-1-5 |
| **c45** | 22 | 42 | 92 | 142 | 148 | 182 | PN105 | 24E10-4 |
| **c46** | 23 | 43 | 89 | 140 | 148 | 182 | PN106 | 20G6-7-1 |
| **c47** | 23 | 45 | 77 | 141 | 148 | 168 | PN107 | 21F3-11 |
| **c48** | 23 | 42 | 87 | 136 | 148 | 185 | PN108 | 26G9-9 |
| **c49** | 23 | 42 | 83 | 141 | 148 | 186 | PN109 | 24A12-12 |
| **c50** | 26 | 43 | 114 | 143 | 155 | 187 | PN110 | 29B8-3 |
| **c51** | 17 | 42 | 91 | 145 | 148 | 188 | PN111 | 17D5-11 |
| **c52** | 23 | 42 | 93 | 118 | 157 | 189 | PN112 | 26C3-4 |
| **c53** | 37 | 53 | 72 | 129 | 158 | 190 | PN113 | 55H6-6 |
| **c54** | 22 | 42 | 92 | 141 | 148 | 183 | PN114 | 18F5-2 |
| **c55** | 7 | 65 | 70 | 136 | 148 | 191 | PN115 | 24F10-1 |
| **c56** | 32 | 54 | 81 | 124 | 153 | 174 | PN116 | 23D1-8 |
| **c57** | 13 | 49 | 101 | 140 | 148 | 182 | PN117 | 20D2-12 |
| **c58** | 23 | 42 | 84 | 137 | 148 | 168 | PN118 | 24B9-10 |
| **c59** | **10** | **64** | **108** | **116** | **154** | **192** | **PN119** | **35B8-6** |
| **c60** | 14 | 61 | 73 | 140 | 148 | 182 | PN120 | 14D3-7-3 |
| **c61** | 20 | 43 | 78 | 131 | 148 | 193 | PN121 | 28E3-4 |
| **c62** | 15 | 62 | 95 | 123 | 159 | 194 | PN122 | 20G2-3-7 |
| **c63** | **9** | **58** | **96** | **120** | **155** | **195** | **PN123** | **38D9-12** |
| **c64** | 25 | 42 | 106 | 138 | 148 | 196 | PN124 | 18F11-4 |
| **c65** | **13** | **47** | **100** | **130** | **147** | **172** | **PN125** | **21D2-3** |
| **c66** | 16 | 44 | 103 | 141 | 148 | 197 | PN126 | 15A7-6 |
| **c67** | 32 | 54 | 97 | 136 | 148 | 185 | PN127 | 30B1-3 |
| **c68** | **19** | **42** | **80** | **122** | **148** | **198** | **PN128** | **1F10-12-3** |
| **c69** | **23** | **43** | **78** | **115** | **148** | **193** | **PN129** | **4D9-12** |
| **c70** | 29 | 51 | 68 | 118 | 155 | 199 | PN130 | 48D10-5 |
| **c71** | 8 | 42 | 113 | 141 | 148 | 182 | PN131 | 29A9-5 |

In one or more embodiments, the anti-OX40 antibody of the description contains HCDR and LCDR selected from any of the following groups in Table 3: c3, c5, c11, c16, c19, c20, c21, c41, c59, c63, c65, c68, c69.

The FR1 of the anti-OX40 antibody VH of the description can be selected from the FR1 of the VH with each antibody number in Table 4, FR2 from the FR2 of the VH with each antibody number in Table 4, FR3 from the FR3 of the VH with each antibody number in Table 4, and FR4 from the FR4 of the VH with each antibody number in Table 4; FR1 of and/or VL can be selected from FR1 of VL with antibody number in Table 4, FR2 from FR2 of VL with antibody number in Table 4, FR3 from FR3 of VL with antibody number in Table 4, and FR4 from FR4 of VL with antibody number in Table 4.

**Table 4**

| Group | Recombinant antibody number | Name of corresponding hybridoma antibody (if any) | VH | VL |
|---|---|---|---|---|
| **d1** | PN001 | | 200 | 271 |
| **d2** | PN002 | | 201 | 272 |
| **d3** | PN003 | | 202 | 273 |
| **d4** | PN004 | | 203 | 274 |
| **d5** | PN005 | | 204 | 275 |
| **d6** | PN006 | | 205 | 276 |
| **d7** | PN008 | | 206 | 277 |
| **d8** | PN009 | | 207 | 278 |
| **d9** | PN011 | | 208 | 279 |
| **d10** | PN012 | | 209 | 280 |
| **d11** | PN013 | | 210 | 281 |
| **d12** | PN014 | | 211 | 282 |
| **d13** | PN017 | | 212 | 283 |
| **d14** | PN019 | | 213 | 284 |
| **d15** | PN021 | | 214 | 285 |
| **d16** | PN024 | | 215 | 286 |
| **d17** | PN027 | | 216 | 287 |
| **d18** | PN028 | | 217 | 288 |
| **d19** | PN029 | | 218 | 289 |
| **d20** | PN03 5 | | 219 | 290 |
| **d21** | PN037 | | 220 | 291 |
| **d22** | PN038 | | 221 | 292 |
| **d23** | PN043 | | 222 | 293 |
| **d24** | PN045 | | 223 | 294 |
| **d25** | PN046 | | 224 | 295 |
| **d26** | PN049 | | 225 | 296 |
| **d27** | PN051 | | 226 | 297 |
| **d28** | PN053 | | 227 | 298 |
| **d29** | PN059 | | 228 | 299 |
| **d30** | PN061 | | 229 | 300 |
| **d31** | PN062 | | 230 | 301 |
| **d32** | PN063 | | 231 | 302 |
| **d33** | PN064 | | 232 | 303 |
| **d34** | PN065 | | 233 | 304 |
| **d35** | PN066 | | 234 | 305 |
| **d36** | PN067 | | 235 | 306 |
| **d37** | PN068 | | 236 | 307 |
| **d38** | PN069 | | 237 | 308 |
| **d39** | PN070 | | 238 | 309 |
| **d40** | PN071 | | 239 | 310 |
| **d41** | PN101 | 16E4-10-5 | 240 | 311 |
| **d42** | PN102 | 24F8-7 | 241 | 312 |
| **d43** | PN103 | 21F9-12 | 242 | 313 |
| **d44** | PN104 | 23C4-1-5 | 243 | 314 |
| **d45** | PN105 | 24E10-4 | 244 | 315 |
| **d46** | PN106 | 20G6-7-1 | 245 | 316 |
| **d47** | PN107 | 21F3-11 | 246 | 317 |
| **d48** | PN108 | 26G9-9 | 247 | 318 |
| **d49** | PN109 | 24A12-12 | 248 | 319 |
| **d50** | PN110 | 29B8-3 | 249 | 320 |
| **d51** | PN111 | 17D5-11 | 250 | 321 |
| **d52** | PN112 | 26C3-4 | 251 | 322 |
| **d53** | PN113 | 55H6-6 | 252 | 323 |
| **d54** | PN114 | 18F5-2 | 253 | 324 |
| **d55** | PN115 | 24F10-1 | 254 | 325 |
| **d56** | PN116 | 23D1-8 | 255 | 326 |
| **d57** | PN117 | 20D2-12 | 256 | 327 |
| **d58** | PN118 | 24B9-10 | 257 | 328 |
| **d59** | PN119 | 35B8-6 | 258 | 329 |
| **d60** | PN120 | 14D3-7-3 | 259 | 330 |
| **d61** | PN121 | 28E3-4 | 260 | 331 |
| **d62** | PN122 | 20G2-3-7 | 261 | 332 |
| **d63** | PN123 | 38D9-12 | 262 | 333 |
| **d64** | PN124 | 18F11-4 | 263 | 334 |
| **d65** | PN125 | 21D2-3 | 264 | 335 |
| **d66** | PN126 | 15A7-6 | 265 | 336 |
| **d67** | PN127 | 30B1-3 | 266 | 337 |
| **d68** | PN128 | 1F10-12-3 | 267 | 338 |
| **d69** | PN129 | 4D9-12 | 268 | 339 |
| **d70** | PN130 | 48D10-5 | 269 | 340 |
| **d71** | PN131 | 29A9-5 | 270 | 341 |

In the preferred embodiment, the FR region of the anti-OX40 antibody VH of the description is the FR region of any VH selected from the antibody SEQ ID NO: 200-270, and the FR region of VL is the FR region of any VL selected from the antibody SEQ ID NO: 271-341. Further preferably, the HCDR of this kind of antibody is selected from any group from groups a1 to a71, and the LCDR is selected from any group from group b1 to group b71; More preferably, the CDR of such antibodies is selected from any of the aforementioned groups c1 to c71.

In some embodiments, the VH amino acid sequence of the anti-OX40 antibody of the description is shown in any of SEQ ID NO: 200-270, and/or the VL amino acid sequence is shown in any of SEQ ID NO: 271-341. Preferably, the amino acid sequence of VH and VL of the anti-OX40 antibody of the description is shown in any row of Table 3.

In some embodiments, the amino acid sequence of the heavy chain constant region of the antibody of the description is shown in any of SEQ ID NO: 342-345, and/or the amino acid sequence of the light chain constant region is shown in SEQ ID NO: 346 or 347. In some embodiments, the amino acid sequence of the heavy chain constant region of the antibody of group d1-d40 in Table 4 is shown in SEQ ID NO: 345, and/or the amino acid sequence of the light chain constant region is shown in SEQ ID NO: 347. In other embodiments, the amino acid sequence of the heavy chain constant region of the antibody of group d41-d71 in Table 4 is shown in any item of SEQ ID NO: 342-345, and/or the amino acid sequence of the light chain constant region is shown in SEQ ID NO: 346 or 347. In this paper, the heavy chain and light chain constant region of each antibody can use any other heavy chain and light chain constant region in the art without affecting the binding ability of antibody and antigen.

| Group | Recombinant antibody number | Corresponding hybridoma antibody name | Hybridoma antibody CH |
|---|---|---|---|
| **e41** | PN101 | 16E4-10-5 | 343 |
| **e42** | PN102 | 24F8-7 | 342 |
| **e43** | PN103 | 21F9-12 | 342 |
| **e44** | PN104 | 23C4-1-5 | 343 |
| **e45** | PN105 | 24E10-4 | 343 |
| **e46** | PN106 | 20G6-7-1 | 343 |
| **e47** | PN107 | 21F3-11 | 344 |
| **e48** | PN108 | 26G9-9 | 344 |
| **e49** | PN109 | 24A12-12 | 343 |
| **e50** | PN110 | 29B8-3 | 343 |
| **e51** | PN111 | 17D5-11 | 344 |
| **e52** | PN112 | 26C3 -4 | 343 |
| **e53** | PN113 | 55H6-6 | 343 |
| **e54** | PN114 | 18F5-2 | 343 |
| **e55** | PN115 | 24F10-1 | 343 |
| **e56** | PN116 | 23D1-8 | 344 |
| **e57** | PN117 | 20D2-12 | 343 |
| **e58** | PN118 | 24B9-10 | 343 |
| **e59** | PN119 | 35B8-6 | 344 |
| **e60** | PN120 | 14D3-7-3 | 343 |
| **e61** | PN121 | 28E3-4 | 344 |
| **e62** | PN122 | 20G2-3-7 | 344 |
| **e63** | PN123 | 38D9-12 | 344 |
| **e64** | PN124 | 18F11-4 | 342 |
| **e65** | PN125 | 21D2-3 | 344 |
| **e66** | PN126 | 15A7-6 | 344 |
| **e67** | PN127 | 30B1-3 | 343 |
| **e68** | PN128 | 1F10-12-3 | 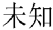 |
| **e69** | PN129 | 4D9-12 | 344 |
| **e70** | PN130 | 48D10-5 | 343 |
| **e71** | PN131 | 29A9-5 | 344 |

The antibody of the description can be chimeric antibody, humanized antibody or complete human antibody; Perfect human antibody is preferred. It should be understood that the antibody provided by the embodiment of the description is a complete human antibody.

Under the premise that the activity of the antibody is not substantially affected, the sequences of the present description can be substituted, added and/or deleted by one or more (eg. 1, 2, 3, 4, 5, 6, 7, 8, 9 or 10 or more) amino acids by those skilled in the art to obtain variants of the antibody or functional fragment sequence thereof. All of them are included in the scope of protection of this description. For example, amino acids with similar properties are substituted in the FR and/or CDR regions of the variable region. Substitutions are preferably conservative substitutions; amino acid residues that can be conservatively substituted are well known in the art. In some embodiments, the variant of this description may be at least 95%, 96%, 97%, 98% or 99% identical to the sequence from which it was derived. Sequence identity according to the present description can be measured using sequence analysis software. For example, the computer programs BLAST using default parameters, especially BLASTP or TBLASTN.

The anti-OX40 antibodies of the description can be modified to affect function. The present description includes anti-OX40 antibodies with modified glycosylation sites. Modifications can be made to remove undesired glycosylation sites, or remove fucose moieties from oligosaccharide chains to enhance antibody-dependent cytotoxicity (ADCC) function, or add galactosylation to alter complement dependent cytotoxicity (CDC).

Anti-OX40 antibodies of the description can typically have affinity constants (KD) of about 10⁻⁹ to about 10⁻¹³ M.

The anti-OX40 antibodies of this description can be prepared by conventional methods in the art, such as hybridoma technology well known in the art. Alternatively, the anti-OX40 antibodies of this description can be expressed in cell lines other than hybridoma cell lines. Suitable mammalian host cells can be transformed with sequences encoding the antibodies of the description. Transformation can be carried out by any known method including, for example, packaging the polynucleotide in a virus (or viral vector) and transducing the host cell with the virus (or vector). The transformation procedure used will depend on the host to be transformed. Methods for introducing heterologous polynucleotides into mammalian cells are well known in the art and include dextran-mediated transfection, calcium phosphate precipitation, polybrene-mediated transfection, protoplast fusion, electroporation, encapsulation of polynucleotides in liposomes and direct microinjection of DNA into the nucleus. Mammalian cell lines that can be used as hosts for expression are well known in the art and include, but are not limited to, various immortalized cell lines available from the American Type Culture Collection (ATCC), including but not limited to Chinese Hamster Ovary (CHO) cells, HeLa cells, baby hamster kidney (BHK) cells, monkey kidney cells (COS), human hepatocellular carcinoma cells (e.g., HepG2), etc. Particularly preferred cell lines are selected by determining which cell lines have high expression levels and produce antibodies with substantial OX40 binding properties.

### Polynucleotide sequences encoding anti-OX40 antibodies

The present description provides nucleic acid molecules comprising polynucleotide sequences encoding the anti-OX40 antibodies of this description. Provided herein are polynucleotide sequences encoding heavy chain variable regions, light chain variable regions, heavy chains, light chains, and each CDR.

DNA and RNA in single- and double-stranded forms, as well as corresponding complementary sequences. DNA includes, for example, cDNA, genomic DNA, chemically synthesized DNA, PCR amplified DNA, and combinations thereof. Nucleic acid molecules of the present description include full-length genes or cDNA molecules and combinations of fragments thereof. Nucleic acids of this description are preferably derived from human sources, but the description also includes nucleic acids derived from non-humans.

In this description, an isolated nucleic acid molecule refers to a nucleic acid molecule in the form of individual fragments or as a component of a larger nucleic acid construct. In a preferred embodiment, the nucleic acid is substantially free of contaminating endogenous material. Nucleic acid molecules are preferably derived from DNA or RNA that has been isolated at least once in substantially pure form and in an amount or concentration that enables identification, manipulation and recovery of its constituent nucleotide sequences by standard biochemical methods. The sequences are preferably provided and/or constructed as open reading frames uninterrupted by internal untranslated sequences or introns (typically found in eukaryotic genes). Sequences of untranslated DNA may be present 5' or 3' to the open reading frame, which again does not affect the manipulation or expression of the coding region.

Nucleic acids that hybridize to nucleic acids encoding anti-OX40 antibodies as described herein under moderately stringent conditions, preferably under highly stringent conditions are included in the description. Basic parameters affecting the selection of hybridization conditions and guidance on designing suitable conditions can be found in Sambrook, Fritsch and Maniatis (1989, Molecular Cloning : A Laboratory Manual, Cold Spring Harbor Laboratory Press, Cold Spring Harbor, NY, Chapters 9 and 11 ; and Current Protocols in Molecular Biology, 1995, edited by Ausubel et al., John Wiley & Sons, Inc., Sections 2.10 and 6.3-6.4).

As outlined herein, cassette mutagenesis or PCR mutagenesis or other techniques well known in the art are typically used for site-specific mutagenesis of nucleotides in DNA encoding anti-OX40 antibodies to generate DNA encoding variants, and thereafter the recombinant DNAs are expressed in cell culture to prepare variants according to the description. However, antigen-binding fragments comprising up to about 100-150 residues can be prepared by in vitro synthesis using established techniques.

As will be appreciated by those skilled in the art, due to the degeneracy of the genetic code, extremely large numbers of nucleic acids can be made, all of which encode the anti-OX40 antibodies or antigen-binding fragments thereof of the description. Thus, having identified a particular amino acid sequence, one of skill in the art can make any number of different nucleic acids by simply modifying the sequence of one or more codons in a manner that does not alter the amino acid sequence encoding the protein.

The present description also provides expression systems and constructs in the form of plasmids, expression vectors, transcription cassettes or expression cassettes comprising at least one polynucleotide as described above. Additionally, the present description provides host cells comprising the expression system or construct.

Expression vectors used in any host cell typically contain sequences for plasmid maintenance and for cloning and expression of exogenous nucleotide sequences. The sequences (collectively referred to in certain embodiments as "flanking sequences") typically include one or more of the following nucleotide sequences: a promoter, one or more enhancer sequences, an origin of replication, a transcription termination sequence, a complete intronic sequence containing donor- and receptor-splice sites, a sequence encoding leader sequence for polypeptide secretion, a ribosome binding site, polyadenylation sequence, a polylinker region for insertion of nucleic acids encoding antibodies to be expressed and optional marker elements. Each of these sequences is discussed below.

The vector may optionally contain a "tag" coding sequence, an oligonucleotide molecule located at the 5' or 3' end of the anti-OX40 antibody coding sequence; the oligonucleotide sequence encoding a polyhistidine (such as 6His) or another "tags", such as FLAG, HA (hemagglutinin influenza virus) or myc, are present in commercially available antibodies. This tag is typically fused to the polypeptide when it is expressed, and can serve as a means for affinity purification or detection of anti-OX40 antibodies from host cells. Affinity purification can be accomplished, for example, by column chromatography using an antibody against this tag as an affinity matrix. The label can be optionally removed later from purified anti-OX40 antibodies by various means such as the use of certain peptidases for cleavage.

Flanking sequences can be homologous (i.e., from the same species and/or strain as the host cell), heterologous (i.e., from a species other than the host cell species or strain), heterozygous (i.e., a combination of flanking sequences from more than one source), synthetic or natural. Similarly, the source of the flanking sequences can be any prokaryotic or eukaryotic organism, any vertebrate or invertebrate organisms or any plant, provided that the flanking sequences are functional and activable by host cell machinery.

The origin of replication is typically part of those prokaryotic expression vectors that are commercially available, and this origin facilitates the amplification of the vector in the host cell. If the chosen vector does not contain an origin of replication site, it can be chemically synthesized and ligated into the vector based on the known sequence. For example, origins of replication from plasmid pBR322 (New England Biolabs, Beverly, MA) are suitable for most Gram-negative bacteria, and various viral origins (e.g., SV40, polyoma, adenovirus, vesicular stomatitis virus (VSV) or papillomavirus, such as HPV or BPV) are suitable for cloning vectors in mammalian cells. Mammalian expression vectors generally do not require an origin of replication (e.g., often only the SV40 origin is used since it also contains the viral early promoter).

Transcription termination sequences are typically located at the 3' end of the polypeptide coding region to terminate transcription. Transcription termination sequences in prokaryotes are usually GC-rich fragments followed by polythymidylate sequences.

The selectable marker gene encodes a protein necessary for the survival and growth of host cells grown in selective media. Typical selectable marker genes encode proteins that (a) provide resistance to antibiotics or other toxins (e.g., ampicillin, tetracycline or kanamycin for prokaryotic host cells); (b) complement the auxotrophy of the cells; or (c) provide important nutrients not available from complexes or defined media. Specific selectable markers are the kanamycin resistance gene, the ampicillin resistance gene, and the tetracycline resistance gene. Advantageously, the neomycin resistance gene can also be used for selection in prokaryotic and eukaryotic host cells.

Ribosome binding sites are often necessary for translation initiation of mRNA and are characterized by Shine-Dalgarno sequences (prokaryotes) or Kozak sequences (eukaryotes). This element is typically located 3' to the promoter and 5' to the coding sequence of the polypeptide to be expressed.

Expression and cloning vectors of the present description will typically contain a promoter recognized by the host organism and operably linked to the anti-OX40 antibody-encoding molecule. A promoter is a non-transcription sequence located upstream of the initiation codon of a structural gene (usually within about 100 to 1000 bp) that controls the transcription of the structural gene.

Suitable promoters for use with yeast hosts are also well known in the art. Yeast enhancers are advantageously used with yeast promoters. Suitable promoters for use with mammalian host cells are well known and include, but are not limited to, some promoters obtained from the viral genomes of such as polyoma virus, fowl pox virus, adenovirus (such as adenovirus 2), bovine papilloma virus, avian sarcoma virus, cytomegalovirus, retrovirus, hepatitis B virus and most preferably simian virus 40 (SV40). Other suitable mammalian promoters include heterologous mammalian promoters, such as heat shock protein (HSP) promoters and actin promoters.

Enhancer sequences can be inserted into vectors to increase transcription by higher eukaryotes of the DNA encoding the light or heavy chains that make up the anti-OX40 antibodies of the description. Enhancers are cis-acting elements of DNA that act on promoters to increase transcription and are usually around 10-300 bp in length. Enhancers are relatively orientation- and position-independent, and enhancers have been found at 5 ' and 3 ' positions in transcriptional units. Several enhancer sequences are known that are available from mammalian genes, such as enhancer sequences of globulin, elastase, albumin, alpha-fetoprotein, and insulin. However, enhancers from viruses are typically used. The SV40 enhancer, cytomegalovirus early promoter enhancer, polyoma enhancer, and adenovirus enhancer known in the art are exemplary enhancer elements for activating eukaryotic promoters.

The expression vector of the present description can be constructed from a starting vector such as a commercially available vector. Such vectors may or may not contain all desired flanking sequences. If one or more of the flanking sequences described herein are not already present in the vector, they can be obtained separately and linked to the vector. Methods for obtaining individual flanking sequences are well known to those skilled in the art.

After constructing the vector and inserting the nucleic acid molecule encoding the light chain, heavy chain, or light and heavy chain of the anti-OX40 antibody into the appropriate sites of the vector, the completed vector can be inserted into a suitable host cell for amplification and/or polypeptide expression. The expression vector for anti-OX40 antibodies can be transformed into host cells of choice by well-known methods including transfection, infection, calcium phosphate coprecipitation, electroporation, microinjection, lipofection, DEAE-dextran mediated transfection or other known techniques. The selected part of the method may vary with the type of host cell to be used.

When the host cells are grown under appropriate conditions such that they synthesize anti-OX40 antibodies, the anti-OX40 antibodies can then be collected from the culture medium (if the host cells secrete them into the culture medium) or directly from the host cells from which they were produced (if not secreted). Suitable host cells are as previously described.

### Anti-QX40 antibodies for therapeutic purposes

All aspects of the anti-OX40 antibody described herein can be used to prepare drugs for the treatment of various conditions and diseases described herein, especially diseases or conditions related to immune cells expressing OX40 (especially T cells, NK cells and neutrophils). In some embodiments, the said condition and disease are T-cell related cancers, including but not limited to: bladder cancer cancer, breast cancer, uterus/cervix cancer, ovarian cancer, prostate cancer, testicular cancer, esophageal cancer, stomach cancer, pancreas cancer, colorectal cancer, colon cancer, kidney cancer, head and neck cancer, lung cancer, stomach cancer, germ cell cancer, bone cancer, liver cancer, thyroid cancer, skin cancer, neoplasms of the central nervous system, lymphoma Leukemia, myeloma, urogenital carcinoma, urothelial carcinoma, renal cell carcinoma, gastric adenocarcinoma, non-small cell lung cancer, diffuse large B-cell lymphoma, head and neck squamous cell carcinoma, Hodgkin's lymphoma, gastroesophageal junction adenocarcinoma, melanoma, sarcoma and virus-related cancer. In some embodiments, cancer is metastatic cancer, refractory cancer or recurrent cancer. The conditions and diseases can also be other OX40-mediated diseases, including, for example, OX40-mediated allergic reactions, asthma, COPD, rheumatoid arthritis, psoriasis, ulcerative colitis, atopic dermatitis, autoimmune diseases and inflammation-related diseases.

### Diagnostic uses, assays and kits

Anti-OX40 antibodies of the description can be used in diagnostic assays, e.g., binding assays to detect and/or quantify OX40 expressed in tissues (such as bone marrow) or cells (such as plasma cells). Anti-OX40 antibodies can be used in further studies investigating the role of OX40 in disease. Anti-OX40 antibodies can be used to further study the function of OX40 in the formation of homo- and/or heteromeric receptor complexes and the function of said OX40 receptor complexes in disease.

The serum level of OX40 can be prognostic. The implementation scheme of the description includes diagnostic determination and kit to measure soluble OX40, which is a potential substitute for membrane-bound OX40 on tumor cells.

The anti-OX40 antibody of the description can be used for diagnostic purposes to detect, diagnose or monitor diseases and/or conditions related to OX40. The present description provides a method for detecting the presence of OX40 in samples using classical immunohistochemical methods known to those skilled in the art. OX40 can be tested in vivo or in vitro. Examples of methods applicable to detecting the presence of OX40 include ELISA, FACS, RIA, etc.

For diagnostic applications, anti-OX40 antibodies are usually labeled with detectable labeling groups. Suitable labeling groups include (but are not limited to) the following: radioisotopes or radionuclides (such as 3H, 14C, 15N, 35S, 90Y, 99Tc, 111In, 125I, 131I), fluorescent groups (such as FITC, rhodamine, lanthanide element phosphors), enzymatic groups (such as horseradish peroxidase, β Root galactosidase, luciferase, alkaline phosphatase), chemiluminescent group, biotinyl group or predetermined peptide epitope recognized by secondary reporter conductor (e.g., leucine zipper pair sequence, binding site for secondary antibody, metal binding domain, epitope tag). In some embodiments, the labeling group is coupled with the anti-OX40 antibody through various length spacer arms to reduce the potential steric hindrance. Various methods for labeling proteins are known in the art and can be used to carry out the present description.

An aspect of the present description provides recognition of cells expressing OX40. In a specific embodiment, the antibody is labeled with a labeled group and the binding of the labeled antibody to OX40 is detected. In another specific embodiment, the combination of antibody and OX40 is detected in vivo. In another specific embodiment, the technology known in the art is used to separate and measure the antibody-OX40.

Another aspect of the description provides the detection of the existence of a test molecule that competes with the antibody of the description to bind OX40. An example of the determination will involve detecting the amount of free antibody in a solution containing a certain amount of OX40 in the presence or absence of test molecules. An increase in the amount of free antibodies (i.e., antibodies that do not bind OX40) will indicate that the test molecule can compete with this antibody to bind OX40. In one embodiment, the antibody is labeled with a labeling group. Alternatively, label the test molecules and monitor the amount of free test molecules in the presence or absence of antibodies.

### Pharmaceutical composition, route of administration

The present description provides pharmaceutical compositions comprising a therapeutically effective amount of one or more anti-OX40 antibodies of the present description together with a pharmaceutically acceptable diluent, carrier, solubilizer, emulsifier, preservative and/or adjuvant.

In certain embodiments, acceptable diluents, carriers, solubilizers, emulsifiers, preservatives and/or adjuvants, etc. in the pharmaceutical compositions are preferably nontoxic to recipients at the dosages and concentrations employed. In certain embodiments, pharmaceutical compositions may contain such substances for improving, maintaining, or preserving, for example, the pH, osmolarity, viscosity, clarity, color, isotonicity, odor, sterility, stability, dissolution, or the rate of release, absorption, or penetration of the composition. These substances are known from the prior art, e.g. see REMINGTON'S PHARMACEUTICAL SCIENCES, 18th Edition, A. R. Genrmo ed., 1990, Mack Publishing Company. The optimal pharmaceutical composition may be determined by the intended route of administration, mode of delivery, and desired dosage.

The pharmaceutical compositions of the present description may be selected for parenteral delivery. Alternatively, the composition may be selected for inhalation or delivery through the digestive tract, such as orally. The preparation of such pharmaceutically acceptable compositions is within the skill of the art.

Other pharmaceutical compositions will be apparent to those skilled in the art, including sustained or controlled release delivery formulations comprising anti-OX40 antibodies. Techniques for formulating a variety of other sustained or controlled delivery modes, such as liposomal vehicles, bioerodible microparticles or porous beads, and depot injections, are also known to those of skill in the art.

Pharmaceutical compositions for in vivo administration are usually provided in the form of sterile formulations. Sterilization is achieved by filtration through sterile filtration membranes. When the composition is lyophilized, it can be sterilized using this method before or after lyophilization and reconstitution. Compositions for parenteral administration may be in lyophilized form or stored in solution. Parenteral compositions are usually presented in containers with sterile access ports, such as intravenous solution strips or vials with a hypodermic needle pierceable stopper.

Once formulated, pharmaceutical compositions are stored in sterile vials as solutions, suspensions, gels, emulsions, solids, crystals, or as dehydrated or lyophilized powders. The formulations can be stored in ready-to-use form or reconstituted prior to administration (e.g., lyophilized). The present description also provides kits for producing single-dose administration units. The kits of the present description may each contain a first container with dried protein and a second container with an aqueous formulation. In certain embodiments of the present description, kits are provided containing single-chamber and multi-chambers pre-filled syringes (e.g., liquid syringes and lyophilized syringes).

The present description also provides methods of treating a patient (especially a patient with T cell-related diseases such as T cell-related cancer and autoimmune diseases) by administering the anti-OX40 antibody or antigen-binding fragment thereof or a pharmaceutical composition thereof according to any of the embodiments of the present description.

As used herein, the terms "patient", "subject", "individual", and "subject" are used interchangeably herein to include any organism, preferably an animal, more preferably a mammal (e.g., rat, mouse, dog, cats, rabbits, etc.), and most preferably humans. "Treatment" refers to the administration of a therapeutic regimen described herein to a subject to achieve at least one positive therapeutic effect (e.g., reduction in cancer cell number, reduction in tumor volume/burden, reduction in the rate of cancer cell infiltration into surrounding organs, or reduction in the rate of tumor metastasis or tumor growth). Treatment regimens that effectively treat a patient can vary depending on factors such as the patient's disease status/stage, age, weight, and the ability of the therapy to elicit an anticancer response in the subject.

The therapeutically effective amount of a pharmaceutical composition containing an anti-OX40 antibody or antigen-binding fragment thereof of the description to be employed will depend on, for example, the extent and objective of treatment. Those skilled in the art will appreciate that the appropriate dosage level for treatment will vary depending in part on the molecule being delivered, the indication, the route of administration and the size (body weight, body surface or organ size) and/or status (age and general health) of the patient. In certain embodiments, the clinician can titrate the dose and alter the route of administration to obtain optimal therapeutic effect.

In certain embodiments, the clinician can titrate the dose and alter the route of administration to obtain optimal therapeutic effect. Clinicians typically administer the compositions until a dosage is reached that achieves the desired effect. The composition may thus be administered as a single dose, or as two or more doses over time (which may or may not contain the same amount of the desired molecule), or as a continuous infusion through an implanted device or catheter.

The route of administration of the pharmaceutical composition is according to known methods, such as oral, intravenous, intraperitoneal, intracerebral (intraparenchymal), intracerebroventricular, intramuscular, intraocular, intraarterial, intra-portal-vein or intralesional route injection; sustained release systems or via implanted devices.

Embodiments included herein
1. An anti-OX40 antibody or an antigen-binding fragment thereof, wherein the anti-OX40 antibody contains HCDR1 as set forth in SEQ ID NO: 1, HCDR2 as set forth in SEQ ID NO:2, and HCDR3 as set forth in SEQ ID NO:3, and/or LCDR1 as set forth in SEQ ID NO:4, LCDR2 as set forth in SEQ ID NO:5, and LCDR3 as set forth in SEQ ID NO: 6.
2. The anti-OX40 antibody or the antigen-binding fragment thereof of item 1, wherein the anti-OX40 antibody comprises HCDR1 as set forth in any of SEQ ID NO:7-38, HCDR2 as set forth in any of SEQ ID NO:39-65, and HCDR3 as set forth in any of SEQ ID NO:66-114, and/or comprises LCDR1 as set forth in any of SEQ ID NO: 115-145, LCDR2 as set forth in any of SEQ ID NO: 146-159, and LCDR3 as set forth in any of SEQ ID NO: 160-199.
3. The anti-OX40 antibody or the antigen-binding fragment thereof of item 1, wherein the anti-OX40 antibody comprises HCDR1, HCDR2 and HCDR3 as set forth in any group from group a1 to group a71 in Table 1, and/or LCDR1, LCDR2 and LCDR3 as set forth in any group from group b1 to group b71 in Table 2.
4. The anti-OX40 antibody or the antigen-binding fragment thereof of item 1, wherein the anti-OX40 antibody comprises HCDR and LCDR as set forth in any group from group c1 to group c71 in Table 3.
5. The anti-OX40 antibody or the antigen-binding fragment thereof of any one of items 1-4, wherein the FR regions of the anti-OX40 antibody VH are FR regions of any VH selected from the VHs shown in SEQ ID NO: 200-270, and the FR regions of the VL are FR regions of any VL selected from the VLs shown in SEQ ID NO: 271-341; More preferably, each FR region of the anti-OX40 antibody VH and VL is the FR region of the VH and VL of any antibody selected from the antibodies in Table 4.
6. The anti-OX40 antibody or the antigen-binding fragment thereof of item 1, wherein the amino acid sequence of VH of the anti-OX40 antibody is as set forth in any of SEQ ID NO: 200-270, and/or the amino acid sequence of VL is as set forth in any of SEQ ID NO: 271-341; Preferably, the amino acid sequence of VH and VL of the anti-OX40 antibody is as set forth in any antibody number in Table 4.
7. The anti-OX40 antibody or the antigen-binding fragment thereof of any one of items 1-6, wherein the anti-OX40 antibody is a chimeric antibody or a complete human antibody; preferably as a complete human antibody.
8. A pharmaceutical composition, wherein the pharmaceutical composition contains the anti-OX40 antibody or the antigen-binding fragment thereof of any one of items 1-7, and a pharmaceutically acceptable excipient or carrier.
9. A nucleic acid molecule or vector containing the nucleic acid molecule, selected from the group consisting of:
   (1) a polynucleotide sequence encoding the anti-OX40 antibody or antigen-binding fragment thereof of any one of items 1-7;
   (2) the complementary sequence of the polynucleotide sequence of (1).
10. Use of the anti-OX40 antibody or the antigen-binding fragment thereof of any one of items 1-7 in manufacture of a medicament for the treatment of T cell-related diseases; preferably, the T cell-related diseases are T cell-related tumors or OX40-mediated diseases; more preferably, the OX40-mediated diseases include OX40-mediated allergic reactions, asthma, COPD, rheumatoid arthritis, psoriasis, autoimmune diseases and inflammation-related diseases.

The present description will be described below in the form of specific embodiments. It should be understood that these embodiments are only illustrative and are not intended to limit the scope of the description. Unless otherwise stated, the methods and materials used in the embodiments are conventional materials and methods in the art.

### Example

### Materials and methods

1) The mice AceMouseGBTM with humanized immunoglobulin variable region gene were obtained from Hunan ACEMAB CORPORATION LIMITED. The mice were raised in Hunan ACEMAB CORPORATION LIMITED. The animal experiments were carried out in accordance with Chinese laws and regulations at all levels, and were approved by the Animal Care and Use Committee of Hunan ACEMAB CORPORATION LIMITED.
2) OX40 humanized mice (hOX40^{Tg} mice): human-mouse chimeric Tnfrsf4 (OX40) cDNA -wpre-polyA (wherein the extracellular region (about 2.3kb) and the first half of the transmembrane region of OX40 gene are from human, including the first half of Exon1-5 and Exon6, and the second half of the transmembrane region and the intracellular region are from mouse, including the second half of Exon7 and Exon6) This strain of mouse was constituted by Shanghai Southern Model Biotechnology Co., Ltd. using CRISPR/Cas9 technology. The mice with this genotype are suitable for the study of mouse anti-human OX40 antibodies.
3) Human FCGR^{Tg} mice are mice having humanized Fc receptor (murine FcyRs knock-outed mouse (FcγRα^{-/-}) expressing human FcyR transgenes (hFcγRI⁺/hFcγ RIIA^{R131+}/hFcγRIIB+/hFcγRIIIA^{F158+}/hFcγRIIIB⁺)) (The mouse has been described in Nat Commun. 2019 Sep 27; 10 (1): 4206 Doi: 10.1038/s41467-019-12097-6.). Mice of this genotype express human Fc receptors.
4) FcyR humanized OX40 humanized mice (hFCGR^{Tg}hOX40^{Tg} mice): obtained by hybridizing the above Fc receptor humanized mice with OX40 humanized mice (hOX40^{Tg} mice). The mice of this genotype simultaneously express human Fc receptors and human OX40 molecules, which is suitable for the study of human anti-human OX40 antibodies.
5) hOX40^{Tg} OT1 mice: obtained by hybridization of OT1 mice with OX40 humanized mice (hOX40^{Tg} mice). CD8+T cells of this genotype of mice have OVA specificity and express human OX40 molecules, which is suitable for the study of human anti-human OX40 antibodies.

All mice were raised in the Animal Science Experiment Center of Shanghai Jiaotong University School of Medicine. All animal experiments were carried out in accordance with Chinese laws and regulations at all levels, and were approved by the Animal Care and Use Committee of Shanghai Jiaotong University School of Medicine.

### Example 1: Immunizing mice to produce monoclonal antibodies against human OX40

For the first immunization, commercialized recombinant human OX40 antigen protein (6 His label, product number CK60, Novoprotein) and standard Freund's complete adjuvant were used at the dose of 40 µg of recombinant human OX40 protein antigen per 20 g of mouse body weight. For the second to fourth immunization, mice aged 6 weeks were immunized with a proprietary expression vector expressing human OX40 full-length protein (refer to NCBI database for sequence, No. NM_003327.2). The fifth immunization was carried out with commercial recombinant human OX40 antigen protein (6His label, product number CK60, Novoprotein) and PBS at the dose of 25 micrograms of human OX40 protein antigen per mouse.

### Example 2: enzyme-linked immunosorbent assay on immunized mouse serum

Dilute the recombinant human OX40 antigen protein (Novoprotein, product No. CK60) with PBS to 0.5 ng/µl, add 100µl/well antigen to MaxiSorp^{™} flat-bottom 96 well ELISA plates, block it with a fresh film and place it at 4 °C overnight. After dumping the antigen the next day, wash it with PBS (200µl/well), dry it on absorbent paper after dumping the PBS, and add 200 µl of blocking solution (PBS containing 10% fetal bovine serum) to each well for 2 hours at room temperature. After blocking, drain the blocking liquid and pat it dry on absorbent paper. Add 50 µl of diluent (PBS containing 5% fetal bovine serum) to dilute the serum in a concentration gradient and add the serum to the 96-well plate. After placing at room temperature for 1 hour, drain the sample, wash the plate with washing solution (PBS containing 0.05% Tween-20) for 3 times, and finally drain the washing solution and pat it dry on absorbent paper. Add 50 µl of diluent (PBS containing 5% fetal bovine serum) to dilute the HRP goat anti-mouse IgG secondary antibody (final concentration 0.4 µg/ml; Biogene, Cat No. 405306), and leave it at room temperature for 1 hour, then drain the liquid, wash with washing solution (PBS containing 0.05% Tween-20) for 5 times (200 µl/well), and finally pour the washing solution and pat it dry on absorbent paper. Add 50 µl/well TMB-urea hydrogen peroxide solution (Thermo Scientific^{™}, Cat.No. 34029), put the substrate solution at room temperature in dark for 3-5 minutes, add 50 µl/well 0.25M sulfuric acid to terminate the reaction, and then test the 450nm wavelength light absorption on the multi-function microplate reader.

| Dilution ratio | Mouse 7997 | Mouse 7999 | Mouse 8179 | Mouse 8207 |
|---|---|---|---|---|
| **Preimmunization** | | | | |
| 50 | 0.108 | 0.092 | 0.086 | 0.161 |
| 100 | 0.086 | 0.078 | 0.082 | 0.118 |
| 200 | 0.071 | 0.081 | 0.081 | 0.117 |
| 400 | 0.082 | 0.078 | 0.076 | 0.101 |
| 800 | 0.066 | 0.069 | 0.073 | 0.081 |
| 1600 | 0.136 | 0.07 | 0.149 | 0.178 |

| **1st** | | | | |
|---|---|---|---|---|
| 50 | 2.234 | 1.981 | 2.13 | 1.17 |
| 100 | 2.032 | 1.644 | 2.079 | 0.592 |
| 200 | 1.828 | 1.21 | 1.761 | 0.396 |
| 400 | 1.214 | 0.803 | 1.358 | 0.285 |
| 800 | 0.863 | 0.476 | 0.862 | 0.198 |
| 1600 | 0.577 | 0.313 | 0.601 | 0.167 |

| **2nd** | | | | |
|---|---|---|---|---|
| 500 | 2.092 | 1.922 | 1.873 | 1.606 |
| 1000 | 2.144 | 1.634 | 1.168 | 0.879 |
| 2000 | 2.13 | 1.341 | 0.767 | 0.588 |
| 4000 | 1.674 | 0.799 | 0.372 | 0.3 |
| 8000 | 1.497 | 0.564 | 0.218 | 0.214 |
| 16000 | 0.946 | 0.28 | 0.116 | 0.114 |
| 32000 | 0.719 | 0.201 | 0.087 | 0.094 |

| **3rd** | | | | |
|---|---|---|---|---|
| 2000 | 2.21 | 1.6 | 0.977 | 1.199 |
| 4000 | 1.923 | 1.028 | 0.479 | 0.623 |
| 8000 | 1.814 | 0.835 | 0.371 | 0.492 |
| 16000 | 1.491 | 0.547 | 0.223 | 0.299 |
| 32000 | 1.257 | 0.4 | 0.165 | 0.304 |
| 64000 | 0.862 | 0.25 | 0.111 | 0.147 |
| 128000 | 0.759 | 0.224 | 0.108 | 0.14 |

### Example 3: Construction of recombinant monoclonal antibody by single cell sequencing

After the mice with humanized IgG variable region were immunized with His-labeled human OX40 extracellular segment protein (hOX40-EC-His, Novoprotein, Cat. No. CK60, Lot. No. 0331348), the mouse spleen, inguinal and popliteal lymph node cells were selected for flow cytometry, and the IgD-negative B-lineage cells were selected for single-cell sequencing (10x Genomics). Select the target sequence (light chain variable region and heavy chain variable region sequence) from many sequences according to the characteristics of antigen-specific B cell clone expansion after immunization, and construct it into a complete recombinant antibody having constant region (light chain is Cκ (SEQ ID NO: 347), the heavy chain constant region is the IgG constant region SEQ ID NO: 345). The antibody sequence was inserted into the expression vector and transferred into suspension 293 cells in the form of co-transfection. The supernatant was collected and purified with protein G beads. The purified antibody was separated from the polymer by Size Exclusion Chromatography (SEC), and the monomer antibody was stored in a refrigerator at 4°C or - 20°C for use.

### Example 4: Determination of binding ability of single cell sequencing antibody to hOX40-EC by ELISA

1) 100 µl of hOX40-EC-His (Novoprotein) was coated in a MaxiSorp^{™} flat-bottom 96 well ELISA plate (nunc) at a concentration of 2 µg / ml, and incubated overnight in a fourth-degree refrigerator.
2) Discard the liquid in the plate, wash the plate twice with PBST (PBS containing 0.05% Tween 20), and add 200 µl of 1% BSA (resuspended in PBS), block at room temperature for 2 hours;
3) The purified single cell sequencing antibody, negative control antibody Ctrl hIgG and positive control antibody IBI101 and Pogalizumab were diluted properly (3.16 µg / ml -1 ng/ml). 100 µl of each was added into the blocked well (washed with PBST for 3 times, and discarded liquid) and incubated at room temperature for 1 hour.
4) Wash the plates with PBST for 4 times, pat plates to remove the liquid , and add 100 µl of detecting antibody (goat anti-human IgG Fc HRP (1:100000, Bethyl)) to each well, incubate at room temperature for 1 hour;
5) Wash the plates with PBST for 5 times, pat plates to remove the liquid , add 100 µl of TMB, substrate of HRP, to each well (liquid A and liquid B mixed in equal volume, KPL); After 10-30 minutes of color development, record the OD650 signal value with a microplate reader.

As shown in FIGS. 1 and 6A-B, the single cell sequencing recombinant antibody has OX40 binding ability.

### Example 5: Obtaining hybridoma cells from immunized mouse spleen cells by electrofusion

Recovery SP2/0 mouse myeloma cells in advance; Count SP2/0 mouse myeloma cells on the day of fusion; The spleen of OX40 mice after successful immunization was placed in the transport medium HB medium (containing 1% P/S) and the cells in the isolated spleen were immediately extracted for counting; Mix 1.5×10⁸ spleen cells with 1.5×10⁸ SP2/0 mouse myeloma; Centrifuge 200G for 5 minutes after mixing, discard the supernatant, and wash twice with 20ml fusion solution for standby; Centrifuge at 200G room temperature for 5 minutes, discard the supernatant and add 6.4 ml of electrofusion buffer to resuspend the precipitation; Prepare a 50 ml centrifuge tube and add 13 ml of preheated HB medium; Add the mixed cell suspension into the CUY497X10 electrode which has been sterilized with 75% alcohol and dried, and use the standard operation process of ECFG21 electrofusion instrument (NEPAGENE, model ECFG21) for cell electrofusion; After fusion, suck out the cells and put them into 13 ml of HB medium preheated in the incubator; Resuspend cells with HAT medium, and add the cells into 96 flat plates at 5×10⁵ cells/well ; Place the 96-well plate in a 37°C incubator for static culture, observe the cells every day, and take the supernatant for ELISA screening on the 10th day.

### Example 6: Screening of hybridoma anti-human OX40 monoclonal antibody by enzyme-linked immunosorbent reaction

Dilute OX40 antigen (Novoprotein cat: CK60) with PBS to 0.5 ng/µl; Add 100µl/well antigen to MaxiSorp^{™} flat-bottom 96 well ELISA, block it with fresh film and place it at 4 °C overnight; After draining the antigen the next day, wash the plate with PBS (200µl/well), dry it on absorbent paper after draining the PBS, and add 200µl blocking solution to each well for 2 hours at room temperature; After blocking, drain the blocking liquid and pat it dry on absorbent paper; Add 50 µl of sample (the culture supernatant of OX40 hybridoma cells obtained in Example 5), put it at room temperature for 1 hour, then drainthe sample, wash the plate with washing solution for 3 times, and finally drain the washing solution and pat the plate dry on absorbent paper; Add 50 µl of HRP goat anti-mouse IgG secondary antibody diluted with diluent (PBS containing 5% fetal bovine serum) and put it at room temperature for 1 hour, then drain the liquid, wash the plate with washing solution for 5 times (200 µl/well), finally drain the washing solution and pat it dry on absorbent paper; Add 50 µl/pore TMB-urea hydrogen peroxide solution substrate solution and place it in dark at room temperature for 3-5 minutes, add 50 µl/well of 0.25M sulfuric acid to terminate the reaction, and then detect the 450nm wavelength light absorption on the multi-function microplate reader. The results are shown in the following table:

| Sample name | stoste | Dilute 100 times | Sample name | Dilute 50 times |
|---|---|---|---|---|
| 4D9-12 | 2.386 | 1.261 | 14D3-7-3 | 2.012 |
| 15A7-6 | 2.248 | 1.402 | 19C12-9-6 | 2.121 |
| 21D2-3 | 2.03 | 1.375 | 20G6-7-1 | 2.205 |
| 21F3-11 | 2.578 | 2.609 | 23C4-1-5 | 2.627 |
| 23D1-8 | 2.482 | 2.021 | 16E4-10-5 | 2.532 |
| 24A12-12 | 2.807 | 2.796 | 1F10-3-1 | 1.254 |
| 24B9-10 | 2.191 | 0.574 | 1F10-12-3 | 2.174 |
| 24F8-7 | 2.4 | 2.139 | | |
| 24F10-1 | 2.414 | 2.201 | | |
| 26G9-9 | 2.42 | 2.253 | | |
| 28E3-4 | 2.27 | 2.256 | | |
| 29A9-5 | 1.928 | 0.878 | | |
| 29B8-3 | 2.459 | 2.359 | | |
| 38D9-12 | 2.244 | 2.098 | | |
| 55H6-6 | 2.426 | 1.898 | | |
| 48D10-5 | 2.45 | 2.352 | | |
| 16G2-10 | 2.185 | 2.479 | | |
| 22E6-11 | 2.371 | 2.427 | | |
| 25F4-7 | 2.304 | 2.087 | | |
| 25F4-10 | 2.471 | 2.132 | | |
| 24E10-4 | 2.508 | 2.395 | | |
| 26C3 -4 | 2.322 | 1.908 | | |
| 30B1-3 | 2.311 | 1.889 | | |
| 17D5-11 | 2.455 | 2.318 | | |
| 18F5-2 | 2.346 | 2.203 | | |
| 18F11-4 | 1.868 | 1.321 | | |
| 20D2-12 | 3.813 | 2.319 | | |
| 22D6-3 | 1.874 | 2.296 | | |
| 27C8-12 | 2.612 | 2.067 | | |
| 35B8-6 | 1.467 | 0.215 | | |
| 21F9-12 | 2.365 | 1.998 | | |

In addition, the light chain and heavy chain variable region sequences of the hybridoma antibodies were obtained, and constructed into a complete antibody with a constant region. See Example 3 for the construction method. The heavy chain constant region of the hybridoma antibodies expressed by sample number or sample name is shown in SEQ ID NO: 342-344, and the light chain constant region is shown in SEQ ID NO: 346; The heavy chain constant region of the recombinant antibody expressed by the antibody number with PN is shown in SEQ ID NO: 345, and the light chain constant region is shown in SEQ ID NO: 347.

### Example 7: Detection of anti-human OX40 antibody binding to OX40 over-expressed cell surface OX40 by flow cytometry

After collecting OX40 over-expressed cells by 200G centrifugation, wash them with PBS containing 3% FCS, and then resuspend them in 2.5 ml of PBS containing 3% FCS. Add the cells into 96-well plates with 25 µl of cells per well (2.5×10⁵), add 75µl of samples each (the culture supernatant of OX40 hybridoma cells obtained in Example 5), and add 75µl of anti-OX40 antibody (clone ACT35, Biogene, the final concentration of 1 µg/ml) into the positive reference well; Add 75µl IgG2a isotype control (clone MG2a-53, Biogene, final concentration 1 µg/ml) into the negative reference Well 1; Add 75µl PBS containing 3% FCS into the negative reference well 2, incubate it in a refrigerator at 4°C for 1 hour, and then wash it twice with PBS containing 3% FCS; After the supernatant is drained, the sample well, the positive reference well, the negative reference well 1 and the negative reference well 2 are added with 50µl of second antibody (Ebioscience, goat anti-mouse IgG-PE, article No. 12-4010-82) at the concentration of 500 ng/ml; After that, the plate was incubated in a refrigerator at 4 °C in dark for 30 minutes, and then washed twice with PBS containing 3% FCS. Finally, the cells were resuspended in 50 µl of PBS containing 3% FCS for detection by flow cytometry. The results are shown in the following table and Figure 2A-C.

| **Clone Name** | **28E3-4** | **21F3-11** | **29B8-3** | **24F10-1** | **55H6-6** |
|---|---|---|---|---|---|
| Y586 channel PE fluorescence value | 195411 | 170723 | 388242 | 274205 | 479073 |

| **Clone Name** | **24E10-4** | **30B1-3** | **15A7-6** | **22D6-3** | **23D1-8** |
|---|---|---|---|---|---|
| Y586 channel PE fluorescence value | 343778 | 208478 | 180959 | 461976 | 166107 |

| **Clone Name** | **24B9-10** | **24A12-12** | **29A9-5** | **26G9-9** | **35B8-6** |
|---|---|---|---|---|---|
| Y586 channel PE fluorescence value | 313454 | 343068 | 220618 | 221755 | 243286 |

| **Clone Name** | **38D9-12** | **4D9-12** | **48D 10-5** | **18F11-4** | **18F5-2** |
|---|---|---|---|---|---|
| Y586 channel PE fluorescence value | 243343 | 186812 | 358123 | 402043 | 431911 |

| **Clone Name** | **26C3-4** | **25F4-10** | **25F4-7** | **16G2-10** | **22E6-11** |
|---|---|---|---|---|---|
| Y586 channel PE fluorescence value | 416371 | 612396 | 616613 | 475068 | 549525 |

| **Clone Name** | **17D5-11** | **27C8-12** | **21F9-12** | **23C4-1-5** | **16E4-10-5** |
|---|---|---|---|---|---|
| Y586 channel PE fluorescence value | 475068 | 400528 | 423614 | 63905 | 376890 |

| **Clone Name** | **1F10-3-1** | **14D3-7-3** | **1F10-12-3** | **20G6-7-1** | **19C12-9-6** |
|---|---|---|---|---|---|
| Y586 channel PE fluorescence value | 172510 | 273719 | 169911 | 351786 | 315714 |

| **Clone Name** | **20D2-12** | **24F8-7** | **21D2-3** | **anti-OX40 Ab** | **IgG2a** |
|---|---|---|---|---|---|
| Y586 channel PE fluorescence value | 540481 | 437030 | 201913 | 209583 | 1828 |

| **Clone Name** | **PBS** | | | | |
|---|---|---|---|---|---|
| Y586 channel PE fluorescence value | 1388 | | | | |

### Example 8: Detection of the binding ability of anti-hOX40 mouse antibodies in hybridoma supernatant to hOX40-EC by ELISA

1) Coat 100 µl of hOX40-EC-His (Novoprotein) in a 96-well high-adsorption ELISA plate (nunc) at a concentration of 1µg/ml, and incubate overnight in a fourth-degree refrigerator;
2) Discard the liquid in the plate, wash the plate twice with PBST (PBS containing 0.05% Tween 20), add 200µl of 1% BSA (resuspended in PBS), and block at room temperature for 2 hours;
3) Dilute hybridoma supernatants (1:10/1:100/1:1000/1:10000), take 100 µl of each into the above-mentioned blocked wells (wash 2 times with PBST, discard the liquid), and incubate at room temperature for 1 hour;
4) Wash the plate 4 times with PBST, pat to remove the liquid, add 100 µl of detection antibody goat anti-mouse IgG Fc HRP (1:5000, Jackson ImmunoResearch) to each well and incubate at room temperature for 1 hour;
5) Wash the plate 5 times with PBST, pat to remove the liquid, add 100 µl of HRP substrate TMB (solution A and solution B mixed in equal volume , KPL) to each well, and record the OD650 signal value with a microplate reader after a period of time;
6) The signal values of the 1: 100 diluted samples was selected for analysis.

The results are shown in the following table and Figure 3 and Figure 6A-B, indicating that hybridoma antibodies or recombinant antibody clones with the same variable region have the ability to bind OX40.

| Sam ple Nom ber | In vitro immune activation activity _PBMC | In vitro immune activation activity _Spleen | In vitro immune activation activity _Spleen | Avera ge activi ty | stan dar d dev iati on | Mean /stand ard deviat ion | IgG ELISA (1:100) | Sample name |
|---|---|---|---|---|---|---|---|---|
| 53 | no sample | 1.1 | 0.7 | 0.9 | 0.3 | 3.4 | 22 | 1F10-3-1 |
| 60 | no sample | 1.0 | 0.7 | 0.9 | 0.2 | 4.7 | 2.3 | 1F10-12-3 |
| 46 | no sample | 0.9 | 0.7 | 0.8 | 0.1 | 5.3 | 1.0 | 22C2-12-3 |
| 77 | 0.5 | 0.9 | 0.8 | 0.8 | 0.2 | 3.7 | 22 | 14E8-12 |
| 59 | 0.6 | 0.9 | 0.7 | 0.7 | 0.2 | 4.3 | 2.3 | 1F10-12-1 |
| 75 | 0.9 | 0.6 | 0.5 | 0.7 | 0.2 | 3.1 | 0.1 | 59C7-9 |
| 62 | no sample | 0.8 | 0.4 | 0.6 | 0.2 | 2.8 | 2.2 | 22A12-11-7 |
| 3 | 0.3 | 0.8 | 0.7 | 0.6 | 0.3 | 2.3 | 2.1 | 15A7-2 |
| 2 | 0.4 | 0.7 | 0.7 | 0.6 | 0.2 | 2.9 | 22 | 23D1-10 |
| 54 | 0.0 | 1.0 | 0.8 | 0.6 | 0.5 | 1.2 | 2.2 | 1F10-3-5 |
| 37 | 0.1 | 0.7 | 0.8 | 0.5 | 0.4 | 1.5 | 2.3 | 4D9-12 |
| 39 | 0.2 | 0.8 | 0.7 | 0.5 | 0.3 | 1.7 | 2.1 | 21D2-3 |
| 28 | 0.1 | 0.9 | 0.6 | 0.5 | 0.4 | 1.3 | 2.1 | 35B8-6 |
| 58 | 0.3 | 0.9 | 0.3 | 0.5 | 0.4 | 1.4 | 2.4 | 11F1-12-12 |
| 70 | 0.3 | 0.9 | 0.4 | 0.5 | 0.3 | 1.6 | 2.3 | 17E11-4-10 |
| 73 | 0.4 | 0.7 | 0.5 | 0.5 | 0.2 | 3.1 | 0.1 | 20H5-12 |
| 26 | 0.0 | 0.8 | 0.6 | 0.5 | 0.4 | 1.2 | 2.4 | 38D9-12 |
| 69 | 0.3 | 0.8 | 0.3 | 0.5 | 0.3 | 1.7 | 2.3 | 19C 12-9-6 |
| 27 | 0.0 | 0.8 | 0.7 | 0.5 | 0.5 | 1.0 | 22 | 35B8-7 |
| 33 | -0.1 | 0.7 | 0.7 | 0.5 | 0.5 | 1.0 | 2.4 | 26G9-3 |
| 71 | 0.2 | 0.8 | 0.4 | 0.5 | 0.3 | 1.4 | 2.3 | 20G6-7-11 |
| 22 | no sample | 0.4 | 0.5 | 0.5 | 0.1 | 8.5 | 2.1 | 21F3-10 |
| 79 | 0.2 | 0.6 | 0.5 | 0.5 | 0.2 | 2.1 | 0.2 | 6H5-3 |
| 24 | 0.1 | 0.6 | 0.7 | 0.4 | 0.3 | 1.4 | 1.9 | 21F3-11 |
| 45 | 0.2 | 0.7 | 0.4 | 0.4 | 0.3 | 1.7 | 22 | 16E4-10-11 |
| 11 | 0.0 | 0.9 | 0.4 | 0.4 | 0.5 | 1.0 | 2.3 | 4D9-6 |
| 47 | -0.1 | 0.8 | 0.6 | 0.4 | 0.5 | 0.9 | 1.9 | 22C2-12-6 |
| 68 | 0.0 | 0.9 | 0.3 | 0.4 | 0.4 | 1.0 | 2.4 | 25G4-1-12 |
| 25 | 0.0 | 0.6 | 0.7 | 0.4 | 0.4 | 1.1 | 2.4 | 38D9-10 |
| 4 | 0.4 | 0.3 | 0.5 | 0.4 | 0.1 | 4.0 | 2.0 | 30B1-3 |
| 21 | 0.1 | 0.5 | 0.6 | 0.4 | 0.3 | 1.6 | 22 | 28E3-6 |
| 30 | 0.4 | 0.5 | 0.3 | 0.4 | 0.1 | 5.0 | 0.1 | 55H6-6 |
| 23 | 0.2 | 0.4 | 0.5 | 0.4 | 0.2 | 2.4 | 2.1 | 28E3-4 |
| 12 | 0.2 | 0.6 | 0.3 | 0.4 | 0.2 | 1.9 | 2.2 | 20D6-1 |
| 32 | 0.1 | 0.6 | 0.4 | 0.4 | 0.2 | 1.5 | 0.2 | 3A0-7 |
| 66 | 0.2 | 0.6 | 0.3 | 0.4 | 0.2 | 2.4 | 2.6 | 17C12-3-10-0 |
| 67 | 0.1 | 0.7 | 0.3 | 0.4 | 0.3 | 1.3 | 2.4 | 17C12-3-12 |
| 55 | 0.1 | 0.7 | 0.3 | 0.4 | 0.3 | 1.2 | 2.2 | 17A12-8-3 |
| 38 | 0.1 | 0.6 | 0.3 | 0.3 | 0.3 | 1.4 | 0.2 | 24B9-10 |
| 72 | 0.1 | 0.5 | 0.5 | 0.3 | 0.2 | 1.4 | 2.2 | 40G6-7-9 |
| 44 | 0.0 | 0.7 | 0.3 | 0.3 | 0.4 | 0.9 | 2.1 | 16E4-1-5 |
| 63 | -0.2 | 0.7 | 0.5 | 0.3 | 0.5 | 0.7 | 2.2 | 14D3-7-3 |
| 56 | 0.0 | 0.5 | 0.5 | 0.3 | 0.3 | 1.3 | 2.2 | 17A12-8-12 |
| 29 | -0.1 | 0.7 | 0.3 | 0.3 | 0.4 | 0.8 | 0.1 | SSH6-3 |
| 36 | 0.5 | 0.1 | 0.4 | 0.3 | 0.2 | 1.5 | 2.0 | 26G9-9 |
| 84 | -0.5 | 0.8 | 0.6 | 0.3 | 0.7 | 0.4 | 0.6 | 12G12-3 |
| 51 | -0.1 | 0.9 | 0.1 | 0.3 | 0.5 | 0.6 | 2.4 | 24G0-4 |
| 6 | 0.1 | 0.4 | 0.4 | 0.3 | 0.2 | 1.6 | 1.9 | 30B 1-12 |
| 9 | 0.1 | 0.3 | 0.6 | 0.3 | 0.2 | 1.2 | 2.3 | 21D2-6 |
| 43 | 0.0 | 0.7 | 0.1 | 0.3 | 0.4 | 0.8 | 2.4 | 23C4-1-5 |
| 61 | -0.1 | 0.6 | 0.3 | 0.3 | 0.3 | 0.9 | 22 | 22A12-11-T01 |
| 57 | 0.2 | 0.3 | 0.4 | 0.3 | 0.1 | 2.5 | 2.5 | 11F1-12-10 |
| 41 | -0.1 | 0.6 | 0.3 | 0.3 | 0.3 | 0.9 | 2.5 | 16G9-1-2 |
| 40 | 0.0 | 0.5 | 0.4 | 0.3 | 0.3 | 1.1 | 2.0 | 18F11-1 |
| 48 | -0.2 | 0.5 | 0.5 | 0.3 | 0.4 | 0.7 | 2.0 | 18F11-2 |
| 1 | 0.1 | 0.5 | 0.2 | 0.3 | 0.2 | 1.5 | 2.4 | 23D 1-8 |
| 49 | -0.1 | 0.5 | 0.3 | 0.3 | 0.3 | 0.8 | 2.5 | 16G9-1-5 |
| 78 | 0.0 | 0.4 | 0.3 | 0.3 | 0.2 | 1.3 | 2.3 | 16G2-10 |
| 50 | -0.1 | -1.2 | 0.6 | -0.2 | 0.9 | -0.2 | 2.5 | 18F9-1 |
| | | | | | | | | |
| Ab-CFS E+ | -0.6 | -8.1 | -4.3 | -4.3 | 3.7 | -1.2 | | |
| aCD 3+α CD2 8 | 1.0 | 1.0 | 1.0 | 1.0 | 0.0 | 33.8 | | |
| aCD 3 | 0.0 | 0.1 | 0.0 | 0.1 | 0.1 | 0.8 | | |
| aCD 3+Ct rl Ab1 | 0.1 | 0.0 | -0.1 | 0.0 | 0.1 | -0.1 | | |
| aCD 3+Ct rl Ab2 | -0.1 | -0.1 | 0.0 | -0.1 | 0.1 | -0.4 | | |

### Example 9: In vitro immunoactivation activity analysis of anti-human OX40 antibody in hybridoma supernatant (PBMC method)

1) Preparation of PBMC suspension: Take out the frozen normal blood donor PBMC (Ficoll isolated peripheral blood mononuclear cells from healthy donors) in a -80 °C refrigerator, and quickly put it in a 37 °C water bath for recovery and thawing. After washing twice with preheated PBS, resuspend cells with an appropriate amount of PBS, and count cells with the modified Neubauer counting plate;
2) CFSE labeled PBMC: take out some cells according to the experimental needs, dilute with PBS to adjust the cell density to (2-4)* 10^7/ml, and the volume less than 5 ml. Add CFSE to the final concentration of 5µM_{∘} Evenly mix and incubate the cells in a 37 °C incubator for 15min. Subsequently, the cells were washed twice with a washing solution containing 9 ml of 5% FBS, centrifuged for 5 min at 400g, and the supernatant was discarded. Add proper amount of primary cell culture medium (RPMI+10% FBS+1% Pen/Strep+1% HEPES+1% sodium pyruvate+0.1% 2ME (final concentration 50uM)) to resuspend cells and count them;
3) Excepter for the controls (untreated, CD3 only, CD3+CD28), the aforementioned CFSE labeled cells were resuspended, at the concentration of 2^{∗}10^6 cells/ml, in the primary cell culture medium containing 0.2 µg/ml of anti-human CD3 (clone: H001, Sino Biological).. According to the experimental design, add 100 µl of the above cells to the flat bottom 96-well cell culture plates, so that the number of cells in each well is 2^{∗}10^5. The untreated group was added with cells not labeled with CFSE and without adding anti-human CD3 or other antibodies. CFSE labeled cells and anti-human CD3 were added to the CD3 only group without adding other antibodies. The CD3+CD28 group was added with CFSE labeled cells and anti-human CD3, and added with concentration of 2 µg/ml of anti-human CD28 antibody (clone: H001, Sino Biological) (after equal volume dilution in step 4, the final concentration of anti- CD3 and CD28 is 0.1 µg/ml and 1µg/ml respectively);
4) Dilute hybridoma supernatant samples with the primary culture medium (1:5), 1 µg/ml of ctrl hIgG and IBI101 were used as the negative and positive control antibodies, respectively. 100µl of each was added to the above-mentioned wells containing anti-human CD3 antibody and CFSE-labeled PBMC. Add 100µl of culture medium to the control group;
5) Place the above cells in a 37 °C incubator for 3 days;
6) Detection of T cell proliferation by flow cytometry: the above-cultured cells were transferred to a 96-well U-bottom plate, washed twice with PBS, centrifuged for 5 minutes, and the supernatant was discarded. The cells were re-suspended in 50µl of FACS buffer (PBS containing 0.5 % of FBS, 2mM of EDTA) containing PE-Cy7 anti-human CD4 (clone: SK3,1:500, BD Pharmingen Pharmingen) and APC anti-human Pharmingen (clone: RPA-T8 (RUO, 1:500, BD Pharmingen^{™}). The cells were incubated on ice for 15 minutes, then washed twice with the PBS buffer, resuspended in 200µl of FACS buffer containing DAPI (0.5µg/ml, Invitrogen), and analyzed by flow cytometry.

As shown in Figure 4, hybridoma antibody clones have in vitro immune activation activity (PBMC system).

### Example 10: In vitro immune activation activity analysis of anti-human OX40 antibody in hybridoma supernatant (spleen cell method)

1) Preparation of single cell suspension of spleen leukocytes of hOX40^{Tg} mice: spleen of hOX40^{Tg} mice was taken under aseptic condition, after grinding, the spleen was lysed with 5 ml of ACK red cell lysis buffer on ice for 5 min, and then 9 ml of PBS was added to terminate the lysis and wash spleen cells. Spleen cells were centrifuged for 5min at 400g, and re-suspended with 2 ml of PBS to obtain the single cell suspension of the spleen. A small amount of single-cell suspension was diluted at 1:100, and the cells were counted using a modified Neubauer counter.
2) CFSE labeled spleen cells: Some cells were taken out according to the experimental requirements and diluted with PBS to adjust the cell density to (2-4) ^{∗}10^7/ml with the volume less than 5 ml. CFSE was added at the final concentration of 5 µM. Mix the cells well and incubate them in an incubator at 37°C for 15 min. Then the cells were washed twice with a soluction containing 9 ml of 5% FBS, centrifuged at 400g for 5min, and the supernatant was discarded. 2 ml of primary cell culture medium (RPMI + 10% FBS +1% Pen/Strep +1% HEPES +1% sodium pyruvate + 0.1% 2ME (final concentration 50uM)) was added to resuspend the cells and counted.
3) Except for the controls (untreated, CD3 only, CD3+CD28), the above CFSE-labeled cells were resuspended, at the concentration of 2^{∗}10^6 cells/ml, in the primary cell culture medium containing 0.2 µg/ml of anti-mouse CD3. According to the experimental design, 100 µl of the above cells were added into the 96-well cell culture plates, so that the number of cells per well was 2^{∗}10^5. In the untreated group, cells not labeled with CFSE were added, without adding anti-mouse CD3 or other antibodies. In the CD3 only group, CFSE-labeled cells and anti-mouse CD3 were added without other antibodies. The CD3+CD28 group was added with CFSE-labeled cells and anti-mouse CD3, and 2 µg/ml of anti-mouse CD28 antibody (after equal volume dilution in Step 4, the final concentrations of anti-mouse CD3 and CD28 were 0.1 µg/ml and 1 µg/ml, respectively).
4) The hybridoma supernatant samples were diluted with primary culture medium (1:4), and 1µg/ml of ctrl hIgG and IBI101 were used as negative and positive controls, respectively. 100µl was taken from each diluted samples and added to the wells with anti-mouse CD3 antibody and CFSE-labeled cells. The control group was added with 100µl of medium;
5) The above cells were cultured in a 37°C incubator for 3 days.
6) T cell proliferation was detected by flow cytometry: The cultured cells were transferred to 96-well U-bottom plates, washed twice with PBS, centrifuged at 500g for 5 min, then the supernatant was removed, and the cells were re-suspended in 50µl of FACS buffer (0.5% FBS, 2mM EDTA PBS) containing anti-PE mouse CD4 (clone :GK1.5, 1:500, BD) and anti-APC mouse CD8a (clone :53-6.7, 1:500, BioLegend) , and incubated on ice for 15 min in the dark. Then the cells were washed twice with PBS buffer and re-suspended in 200µl FACS buffer containing DAPI (0.5µg/ml, Invitrogen), and analyzed by flow cytometry.

Figure 5 shows hybridoma antibody clones with immune activation activity in vitro.

### Example 11: Determination of competitive binding ability of anti-human OX40 antibody to hOX40-EC by ELISA

1) The 96 well high-adsorption ELISA plates (nunc) was coated with 100 µl of hOX40-EC-His (Novoprotein, CK60) at a concentration of 2 µg/ml, and incubated overnight in a four-degree refrigerator.
2) Discard the liquid in the plate, wash the plate twice with PBST (PBS with 0.05% Tween 20), and add 200 µl of 1% BSA (resuspended in PBS), blocked at room temperature for 2 hours;
3) Purified anti-human OX40 antibody and the negative control antibody Ctrl hIgG, human OX40-L-6his (Novoprotein, CJ45) were diluted with 0.2 µg/ml of human OX40-L-mFc (Novoprotein, CW25) to a final concentration of 2 µg/ml (antibody to ligand ratio was 10: 1). 100 µl of each solution was added into the above wells (the plates were washed with PBST for 3 times, the liquid was discarded), and incubated at room temperature for lh.
4) The plates ware washed with PBST for four times, and patted to dry the plates, and then 100 µl of the detecting antibody goat anti-mouse IgGl HRP (1:5000, Jackson ImmunoResearch Laboratories, 115-035-205) was added to each well and incubated at room temperature for lh.
5) The plates were washed with PBST for 5 times, and patted to dry the plates, and then 100µl of HRP substrate TMB (liquid A and liquid B mixed in equal volume, KPL) was added to each well. After the color development for 10-30 minutes, the signal value of OD650 was recorded using a microplate reader.

As shown in Figure 9, some anti-human OX40 antibodies have the ability to compete for binding epitopes of ligands.

### Example 12: Ability of anti-human OX40 antibody to bind hOX40 expressed by hOX40-stably transfected 293T cells

1) **hOX40**-stably transfected 293T cells constitutively expressed human OX40 extracellular segment protein. According to the experimental design, 100 µl of hOX40-stably transfected 293T cells or non-OX40-expression 293T cells were added into 96-well U-bottomedplates (resuspended in PBS), so that the number of cells in each well was 1^{∗}10^6. The supernatant was discarded after centrifugation at 400g for 5 min.
2) Dilution of antibody: use PBS to dilute anti-human OX40 recombinant antibody, Ctrl hIgG (Jackson ImmunoResearch) and anti-mouse OX40 antibody OX86 (hIgGl) to 1µg/ml.
3) The cells were suspended with 50 µl of each aforementioned antibodies and incubated on ice for 15 minutes.
4) Wash twice with PBS, centrifuge 400g for 5 minutes, and discard the supernatant.
5) Flow cytometry analysis of the binding to the reported cells: The cells were re-suspended with anti-human IgG Fab-PE (clone: M1310G05, 1:200, BioLegend) in 50µl of FACS buffer (PBS containing 0.5% FBS, 2 mM EDTA), incubated on ice for 15 min in the dark, then washed the cells twice with PBS buffer, and resuspended in 200µl of FACS buffer containing DAPI (0.5µg/ml, Invitrogen) and analyzed by flow cytometry.

As shown in Figure 7, the anti-human OX40 antibodies have the ability to bind OX40 on stably-transfected cells.

### Example 13: Determination of the ability of anti-human OX40 antibody to bind human, mouse and monkey OX40-EC protein by ELISA

1) 100µl of hOX40-EC-His (Novoprotein, CK60), mouse (mouse) OX40-EC (CK52), rhesus monkey (Rhesus) OX40-EC (sinobiological,90846-C08H) and crab eating monkey (Cynomolgus) OX40-EC (Novoprotein, CB17) were coated in MaxiSorp^{™} flat-bottom 96 well ELISA plate (nunc) at a concentration of 2 µg/ml and incubated overnight in a fourth-degree refrigerator.
2) Discard the liquid from the plate, wash the plate twice with PBST (PBS with 0.05% Tween 20), add 200µl of 1% BSA (resuspended in PBS), block at room temperature for 2 hours.
3) The purified anti-human OX40 antibody and negative control antibody Ctrl hIgG were diluted properly (3.16 µg/ml); 100µl of each were added into the above blocked wells (washed 3 times with PBST, discarded liquid) and incubated at room temperature for 1 hour.
4) Wash the plate 4 times with PBST, pat to remove the liquid, and add 100µl of detection antibody goat anti-human IgG Fc HRP (1:50000, Bethyl, A80-104p) to each well, incubate at room temperature for 1 hour.
5) Wash the plate for 5 times with PBST and pat to remove the liquid. 100µl of HRP substrate TMB (solution A and solution B mixed in equal volume, KPL) was added to each well. After 10-30 minutes of coloration, the signal value of OD650 was recorded by a microplate reader.

As shown in Figure 8, the anti-human OX40 recombinant antibody has the ability to specifically bind human and monkey OX40 protein, and generally does not bind mouse OX40.

### Example 14: Determination of in vitro activity of anti-human OX40 antibody by OX40/NF-xB reporter cell

1) OX40/NF-κB Reporter-HEK293 constitutively expresses human OX40 protein, which, when activated, activates NF-κB signal pathway. As NF-κB response element controls the expression of firefly luciferase gene, OX40 activation can be indirectly indicated by detecting firefly luciferase activity. According to the experimental design, 90µl OX40/NF-κB Reporter-HEK293 cells were added to the 96-well transparent bottom white cell culture flat plate to adjust the number of cells in each well to 8^{∗}10^5, and incubated overnight in the incubator at 37°C.
2) Preparation of human FCGR^{Tg} mouse spleen leukocyte single cell suspension: The spleen of human FCGR^{Tg} mice was taken under aseptic condition. After grinding, the spleen was lysed with 5 ml of red cell lysis buffer ACK on ice for 5 minutes, then 9 ml of PBS was added to stop lysis and wash spleen cells. After centrifuging at 400g for 5min, spleen cells were resuspended with 2 ml of cell medium (DMEM + 10% FBS + 1% non-essential amino acid + 1mM pyruvate sodium + 1%Pen/Strep) to obtain spleen single cell suspension. A small amount of single cell suspension was diluted at 1:100, and the cells were counted with a modified Neubauer counter.
3) Dilution of antibody: anti-OX40 antibody, Ctrl hIgG (Jackson ImmunoResearch) and human OX40-L-mFc (Novoprotein, CW25) were diluted to 0.1 µg/ml with cell culture medium (DMEM + 10% FBS + 1% non-essential amino acid + 1 mM sodium pyruvate + 1%Pen/Strep).
4) After discarding the supernatant of 50µl OX40/NF-κB Reporter-HEK293 cells, 20 µl of human FCGR^{Tg} mouse spleen leukocyte single cell suspension (2* 10^7/ml) and 30 µl of above antibody solution were added along the well wall and incubated in 37°C incubator for 6 hours.
5) 100µl of One-step Luciferase reagent (Promega E6120) was added to each well and shaken for 30 min at room temperature. The luminous intensity was measured by chemiluminescence reader.

As shown in Figure 10, anti-human OX40 antibody has in vitro activity (OX40/NF-κB report cell system).

### Example 15: Analysis of in vitro immune activation activity of anti-human OX40 antibody

1) Preparation of spleen leukocyte single cell suspension of hFCGR^{Tg}hOX40^{Tg} mice: The spleen of hFCGR^{Tg}hOX40^{Tg} mice was taken under aseptic condition. After grinding, the spleen was lysed with 5 ml of red cell lysis buffer ACK on ice for 5 minutes, then 9 ml of PBS was added to stop lysis and wash spleen cells. After centrifuging at 400g for 5 min, spleen cells were resuspended with 2ml of PBS to obtain spleen single cell suspension. A small amount of single cell suspension was diluted at 1:100, and the cells were counted with a modified Neubauer counter;
2) CFSE labeling spleen cells: according to the needs of the experiment, some cells were taken out and diluted with PBS to adjust the cell density to (2-4)^{∗}10^7/ml with the volume less than 5 ml, and CFSE was added at the final concentration of 5 µM. The mixture was incubated in an incubator at 37°C for 15 min after mixing. The cells were then washed twice with a solution containing 5% FBS of 9 ml, centrifuged at 400g for 5min, and the supernatant was discarded. Cells were resuspended by 2ml of primary cell culture medium (RPMI + 10% FBS + 1% Pen/Strep + 1% HEPES + 1% sodium pyruvate + 0.1% 2ME (final concentration 50uM) + 1% L-glutamine + 1% non-essential amino acid) and counted;
3) Except for the controls (untreated, CFSE only, CD3 only, CD3+CD28), the above CFSE labeled cells were resuspended with the primary cell culture medium containing 0.2µg/ml anti-mouse CD3e (clone: 145-2C11, BD Pharmingen^{™}) to adjust the cell density to 2^{∗}10^6 / ml. According to the experimental design, 100µl of the above cells were added to a 96-well cell culture plate, so that the number of cells in each well was 2^{∗}10^5. In the untreated group, cells without CFSE labeling were added without adding anti-mouse CD3 or other antibodies . CFSE labeled cells were added in the only CFSE group without adding anti-mouse CD3 or other antibodies. CFSE labeled cells and anti-mouse CD3 were added in the CD3 only group without adding other antibodies. In CD3+CD28 group, CFSE labeled cells and anti-mouse CD3 were added, and 2µg/ml anti-mouse CD28 antibodies (clone 37.51 (RUO), BD Pharmingen^{™}) were added (after equal volume dilution in step 4, the final concentrations of anti-mouse CD3 and CD28 were 0.1µg/ml and 1µg/ml, respectively).
4) Preparation of antibodies of different concentrations: Anti-human OX40 antibody and Ctrl hIgG (Jackson ImmunoResearch) were gradiently diluted to twice the final concentration by primary cell medium. 100µl were taken and added to the above-mentioned well plates added with anti-mouse CD3 antibody and CFSE labeled spleen cells. The control group was added with 100µl of medium.
5) Place the above cells in a 37°C incubator for 3 days;
6) Detection of T cell proliferation by flow cytometry: the above-cultured cells were transferred to a 96-well U-bottom plate, washed twice with PBS, centrifuged for 5 minutes, and the supernatant was discarded. The cells were re-suspended in 50µl FACS buffer (PBS containing 0.5% FBS and 2mM EDTA) containing PE anti-mouse CD4 (clone: GK1.5,1:500, BD) and APC anti-mouse CD8a (clone: 53-6.7, 1:500, BioLegend) and incubated in ice in the dark for 15 minutes. The cells were then washed twice with PBS buffer and resuspended in 200µl FACS buffer containing DAPI (0.5µg/ml, Invitrogen) and CountBright^{™} Absolute Counting Beads (Life Technologies, 2µl/sample), and analyzed by flow cytometry.

As shown in Figure 11A-D, anti-human OX40 antibodies have immune activation activity in vitro.

### Example 16: Analysis of immune activation activity of anti-human OX40 antibody in vivo

OVA-specific CD8+T cell proliferation model was used to detect the immune activation activity of anti-human OX40 recombinant antibody in vivo. The process is as follows:
1) Spleen cells from OT-IhOX40^{Tg} mice (hOX40^{Tg} OT1) were injected into hFCGR^{Tg}hOX40^{Tg} mice via tail vein at day -1 (1^{∗}10^6 / mouse).
2) On day0, hFCGR^{Tg}hOX40^{Tg} mice were immunized with 5µg/mouse of DEC-OVA protein, and a certain dose of anti-human OX40 antibody and Ctrl hIgG (Jackson ImmunoResearch) by intraperitoneal injection. The grouping and antibody dose are as follows:

| | |
|---|---|
| Group 1: | Ctrl hIgG, 50µg/mouse, n=5 |
| Group 2: | PN116 *, 50µg/mouse, n=3 |
| Group 3: | PN121 *, 50µg/mouse, n=3 |
| Group 4: | PN125 *, 50µg/mouse, n=3 |

3) The mice were sacrificed on day 6, and the spleen was taken under aseptic condition. After grinding, the spleen was lysed on ice with 5 ml of red blood cell lyse buffer ACK for 5 minutes, then 9 ml of PBS was added to terminate the lysis and wash spleen cells. After centrifuging at 400g for 5 min, spleen cells were resuspended with 2 ml of PBS to obtain spleen single cell suspension.
4) The proliferation of T cells was detected by flow cytometry: the above spleen cells were transferred to a 96-well U-bottomplate, centrifuged at 500g for 5 minutes, and the supernatant was removed. The cells were re-suspended in 50 µl of FACS buffer (PBS containing 0.5% FBS, 2mM EDTA) containing PE anti-mouse CD4 (clone: GK1.5,1:500, BD) and APC anti-mouse CD8a (clone: 53-6.7, 1:500, BioLegend) and incubated in the dark on ice for 15 minutes. The cells were then washed twice with PBS buffer and resuspended in 200µl FACS buffer containing DAPI (0.5µg/ml, Invitrogen) and CountBright^{™} Absolute Counting Beads (Life Technologies, 2µl/sample), and analyzed by flow cytometry.

As shown in Figure 12, anti-human OX40 antibody has immune activation activity in vivo.

## Claims

1. An anti-OX40 antibody or an antigen-binding fragment thereof, wherein the anti-OX40 antibody contains HCDR1 as set forth in SEQ ID NO: 1, HCDR2 as set forth in SEQ ID NO:2, and HCDR3 as set forth in SEQ ID NO:3, and/or LCDR1 as set forth in SEQ ID NO:4, LCDR2 as set forth in SEQ ID NO:5, and LCDR3 as set forth in SEQ ID NO: 6.

2. The anti-OX40 antibody or the antigen-binding fragment thereof according to claim 1, wherein the anti-OX40 antibody comprises HCDR1 as set forth in any of SEQ ID NO:7-38, HCDR2 as set forth in any of SEQ ID NO:39-65, and HCDR3 as set forth in any of SEQ ID NO:66-114, and/or comprises LCDR1 as set forth in any of SEQ ID NO: 115-145, LCDR2 as set forth in any of SEQ ID NO: 146-159, and LCDR3 as set forth in any of SEQ ID NO: 160-199.

3. The anti-OX40 antibody or the antigen-binding fragment thereof according to claim 1, wherein the anti-OX40 antibody comprises HCDR1, HCDR2 and HCDR3 as set forth in any group from group a1 to group a71 in Table 1, and/or LCDR1, LCDR2 and LCDR3 as set forth in any group from group b1 to group b71 in Table 2.

4. The anti-OX40 antibody or the antigen-binding fragment thereof according to claim 1, wherein the anti-OX40 antibody comprises HCDR and LCDR as set forth in any group from group c1 to group c71 in Table 3.

5. The anti-OX40 antibody or the antigen-binding fragment thereof according to any one of claims 1-4, wherein the FR regions of the anti-OX40 antibody VH are FR regions of any VH selected from the VHs shown in SEQ ID NO: 200-270, and the FR regions of the VL are FR regions of any VL selected from the VLs shown in SEQ ID NO: 271-341; More preferably, each FR region of the anti-OX40 antibody VH and VL is the FR region of the VH and VL of any antibody selected from the antibodies in Table 4.

6. The anti-OX40 antibody or the antigen-binding fragment thereof according to claim 1, wherein the amino acid sequence of VH of the anti-OX40 antibody is as set forth in any of SEQ ID NO: 200-270, and/or the amino acid sequence of VL is as set forth in any of SEQ ID NO: 271-341; Preferably, the amino acid sequence of VH and VL of the anti-OX40 antibody is as set forth in any antibody number in Table 4.

7. The anti-OX40 antibody or the antigen-binding fragment thereof according to any one of claims 1-6, wherein the anti-OX40 antibody is a chimeric antibody or a complete human antibody; preferably as a complete human antibody.

8. A pharmaceutical composition, wherein the pharmaceutical composition contains the anti-OX40 antibody or the antigen-binding fragment thereof according to any one of claims 1-7, and a pharmaceutically acceptable excipient or carrier.

9. A nucleic acid molecule or vector containing the nucleic acid molecule, selected from the group consisting of:
(1) a polynucleotide sequence encoding the anti-OX40 antibody or antigen-binding fragment thereof according to any one of claims 1-7;
(2) the complementary sequence of the polynucleotide sequence of (1).

10. Use of the anti-OX40 antibody or the antigen-binding fragment thereof according to any one of claims 1-7 in manufacture of a medicament for the treatment of T cell-related diseases; preferably, the T-cell-related diseases are T-cell-related tumors or OX40-mediated diseases; more preferably, the OX40-mediated diseases include OX40-mediated allergic reactions, asthma, COPD, rheumatoid arthritis, psoriasis, autoimmune diseases and inflammation-related diseases.
